# EUROPEAN PATENT APPLICATION

(11) **EP 1 986 009 A1**
(43) Date of publication of application: **29.10.2008**
(21) Application number: 07251762.6
(22) Date of filing: 26.04.2007
(51) Int. Cl.: G01N 33/566, C07K 14/705, C07K 14/52

(54) **Screening method**

(71) Applicant: Active Biotech AB, 220 07 Lund (SE)
(72) Inventor: The designation of the inventor has not yet been filed
(74) Representative: Roques, Sarah Elizabeth

(57) **Abstract**

A method of identifying a compound for use in therapy which modulates the interaction of S100A9 with a ligand comprising:
- determining whether a candidate compound is capable of modulating the interaction of S100A9 with RAGE or the interaction of S100A9 with a TLR (Toll like receptor), or
- determining whether a candidate compound is capable of binding to S 100A9 in a manner which disrupts binding of S100A9 with RAGE or the binding of S100A9 with a TLR,
to thereby identify whether the compound may be used in therapy.

## Description

### Field of the Invention

The present invention relates to novel binding interactions and to identifying a compound for use in therapy in a mammal, including in a human. In particular the compound may be for use in cancer therapy or in therapy of an autoimmune disease or inflammatory condition. Methods are provided for identifying novel S100A9 ligands, and agents that interact with S100A9 are also disclosed.

### Background of the Invention

S100A9, also known as calgranulin B and myeloid related protein-14 (MRP-14), is a calcium-and zinc-binding protein that belongs to the S100 protein family. S100A9 is highly expressed by the myeloid cell lineage and is found in the extracellular milieu during inflammatory conditions. S100A9 forms heterodimers with S100A8, another member of the S100 family. However, S100A9 may also form monomers which executes specific functions. Human S100A9 has a molecular mass of about 13 kDa and is composed of 114 amino acid residues.

The S100A8/A9 protein can bind to endothelium through the interaction of S100A9 with heparan sulphate proteoglycans or of the S100A8/A9 complex with carboxylated N-glycans exclusively expressed by endothelial cells after inflammatory activation. Another receptor that binds different S100 proteins is the receptor for advanced glycation end products (RAGE). Direct binding to RAGE has so far been demonstrated for S100A12, S100B, S100A1, and S100P. The receptor for advanced glycation end products (RAGE) is a member of the immunoglobulin superfamily of cell surface molecules. RAGE consists of an extracellular domain, a single transmembrane spanning domain, and a highly charged cytosolic tail. RAGE signaling contributes to the activation of central cellular pathways which involve p38 or p44/42 MAP kinases, Cdc42/Rac, and NF-κB signaling components, thereby influencing features like cell survival, cell motility, and inflammatory response.

Binding of ligands to the RAGE receptor initiates cellular signals that activate NF-κB, which results in transcription of proinflammatory factors. The mammalian TOLL-like receptors (TLR) are receptors that recognize molecular patterns of pathogens. After the engagement of a pathogenic pattern ligand to TLR, recruitment of adaptor proteins, the transcription factor NF-κB is activated by the receptor-driven signaling cascade. It has been proposed that RAGE and TLR's use similar mechanisms which results in inflammatory reactions.

### Summary of the Invention

The inventors have characterized novel binding interactions of S100A9, i.e. a binding interaction with RAGE and a binding interaction with TLR4. They have also characterized the inhibition of these interactions by a compound which is effective in therapy, and thus developed screening methods to allow identification of compounds for therapy.

Accordingly, the invention provides a method of identifying a compound for use in therapy which modulates the interaction of S100A9 with a ligand comprising:
(i) determining whether a candidate compound is capable of modulating the interaction of S100A9 with RAGE or the interaction of S100A9 with a TLR (Toll like receptor), or
(ii) determining whether a candidate compound is capable of binding to S100A9 in a manner which disrupts binding of S100A9 with RAGE or the binding of S100A9 with a TLR,
to thereby identify whether the compound may be used in therapy.

### Description of the drawings

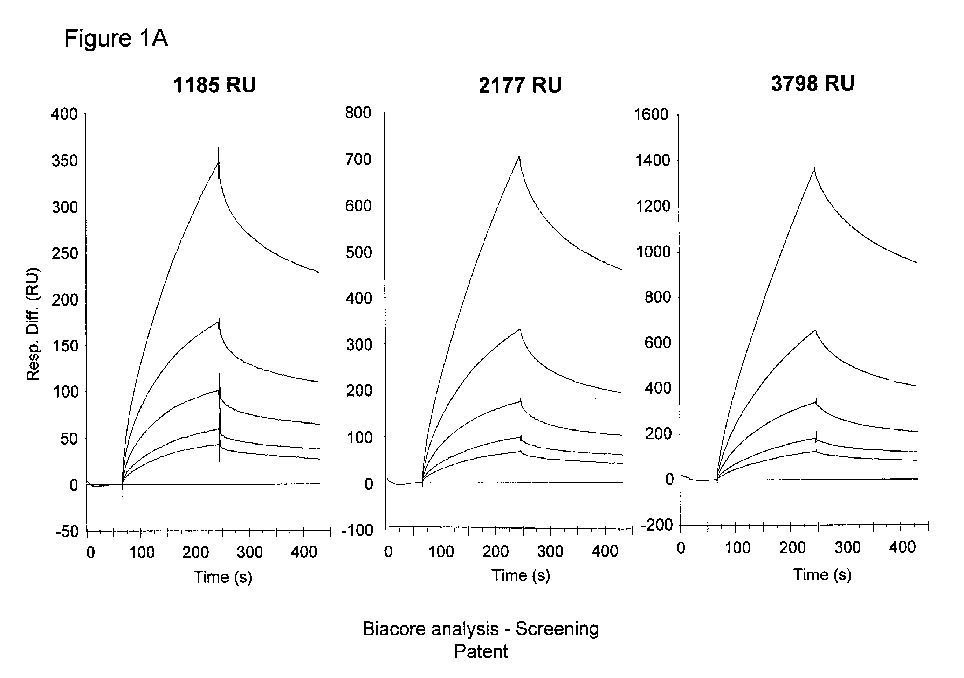

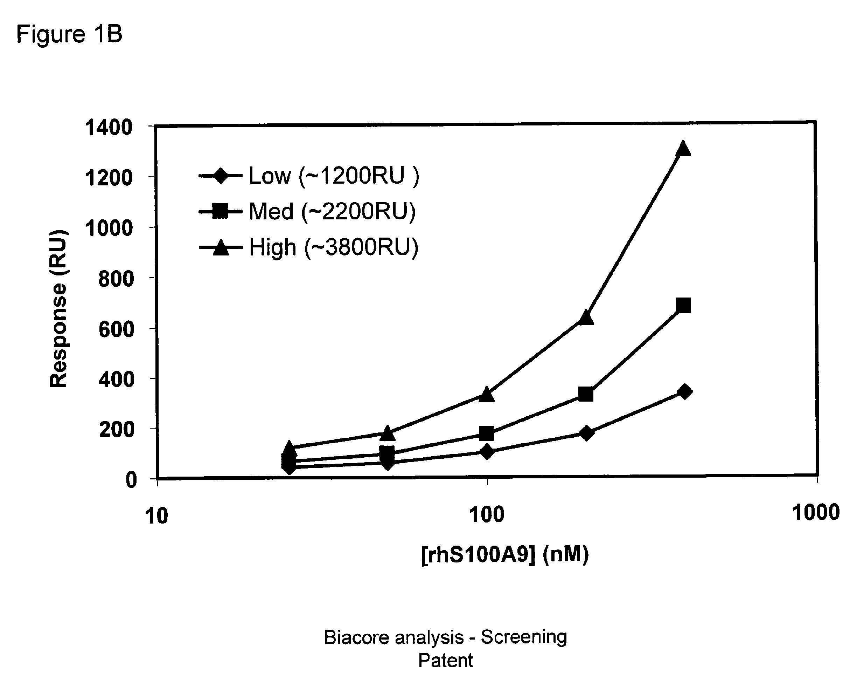

Figure 1 shows binding of 25-400 nM S100A9 to RAGE immobilized by random amine coupling at three densities (approx. 1200, 2200 and 3800 RU in flow cell 2, 3 and 4 respectively). In (A), sensorgrams from bottom to top represent 25, 50, 100, 200 and 400 nM S100A9 after subtraction of the signal in the reference flow cell. Injection time was 3 min at a flow rate of 30 µL/min and regeneration was performed with a 15 µl pulse of 10 mM glycine-HCl, pH 2.0, for 30 s. In (B), responses at late association phase are plotted vs. concentration of S100A9. HBS-P containing 1 mM CaCl₂ and 10 µM ZnCl₂ (Robinson et al. 2002) was used as running and sample buffer.

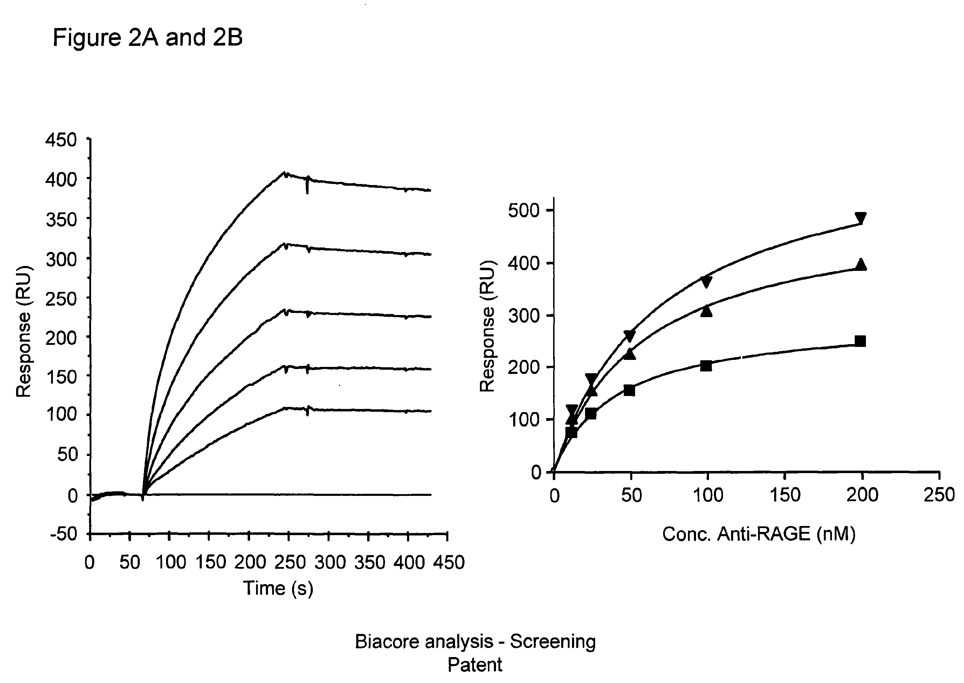

Figure 2 shows a test of activity of immobilized RAGE by injection of a mouse monoclonal antibody recognizing the extra-cellular domain of human RAGE (MAB11451, R&D Systems). In (A), sensorgrams after injection of 12.5-200 nM anti-RAGE over immobilized RAGE (density 2177 RU) are shown and in (B) saturation curves for the three RAGE surfaces in Figure 1 obtained after plotting antibody concentration vs response at late association phase (t ~ 235 s). An apparent dissociation constant, K_{D} of 4.3, 5.8 and 6.9 × 10⁻⁸ M and maximum binding at 295, 502 and 635 RU were calculated for the respective RAGE surfaces after fit of data to a one-site hyperbola binding model (R² > 0.99).

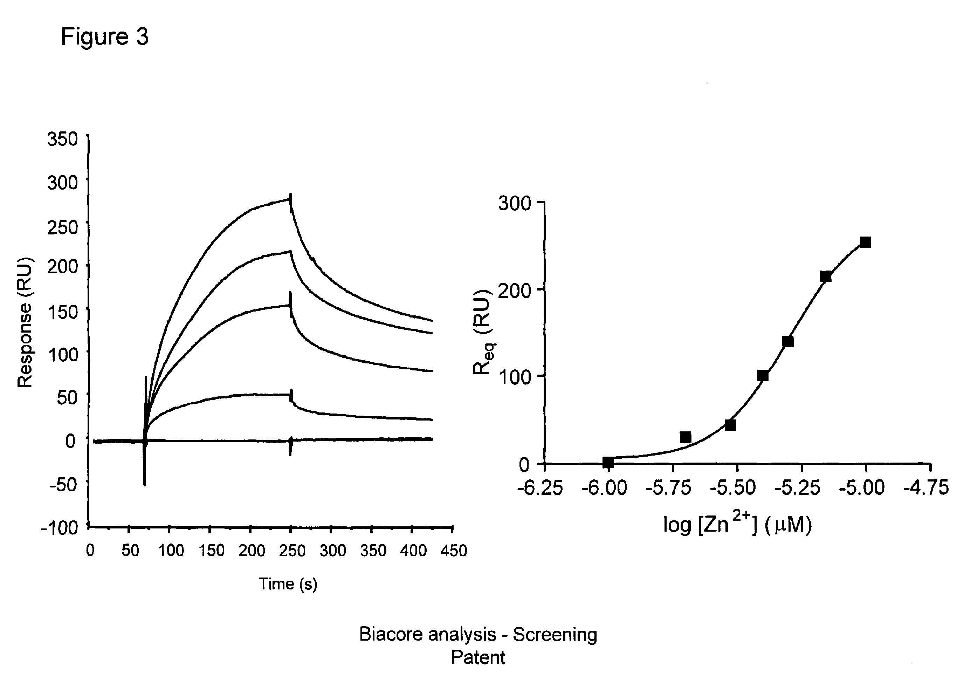

Figure 3 shows titration of Zn²⁺ for optimal binding of 100 nM S100A9 to RAGE. In the left panel, sensorgrams from bottom to top represent: 100 nM S100A9 in HBS-P buffer containing 250 µM CaCl₂ and 0 to 10 µM ZnCl₂. In the right panel, responses at late association phase, R_{eq}, obtained after fitting data to a 1:1 binding model, are plotted vs. the log concentration of Zn²⁺. EC₅₀ was calculated to 5.0 µM Zn²⁺ using a sigmoidal dose-response (R² 0.996) model with variable slope. Experimental conditions were as described in Figure 1.

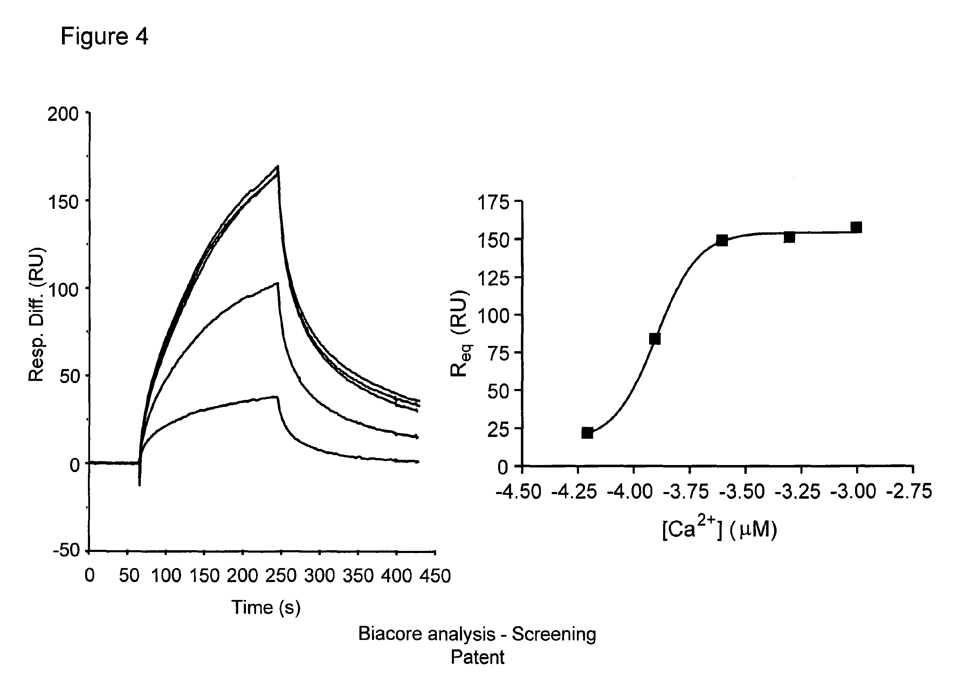

Figure 4 shows titration of Ca²⁺ for optimal binding of 100 nM S100A9 to RAGE. In the left panel, sensorgrams from bottom to top represent: 100 nM S100A9 in HBS-P buffer containing 10 µM ZnCl₂ and 62.5 to 1000 µM CaCl₂. In the right panel, responses at late association phase, R_{eq}, obtained after fitting data to a 1:1 binding model, are plotted vs. the log concentration of Ca²⁺. EC₅₀ was calculated to 126 µM Ca²⁺ using a sigmoidal dose-response (R² 0.999) model with variable slope. Experimental conditions were as described in Figure 1.

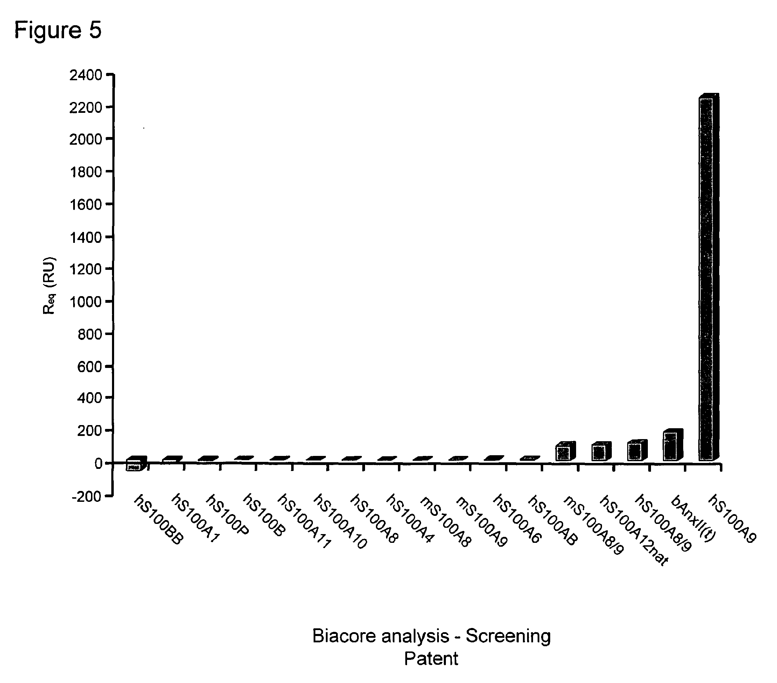

Figure 5 shows binding of 200 nM S100 proteins and annexin II (bovine AnxII₂: S100A10₂ tetrameric complex) to immobilized RAGE. Responses were obtained as R_{eq} values after fit of data to a 1:1 binding model or as the signal at late association phase (t ~ 235 s). Experimental conditions were as in Figure 1. Signals were not corrected for mass contribution.

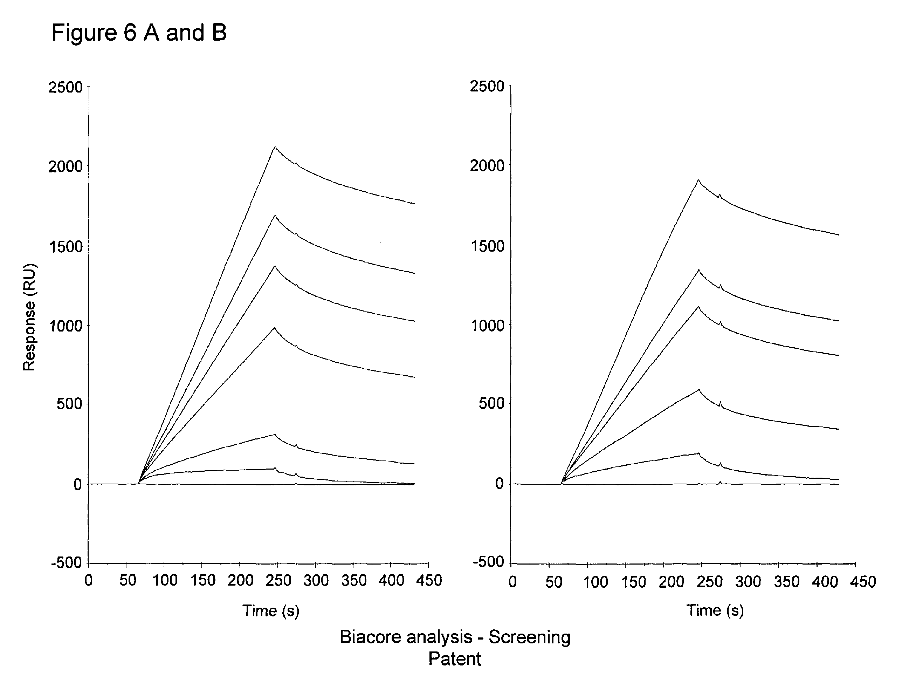

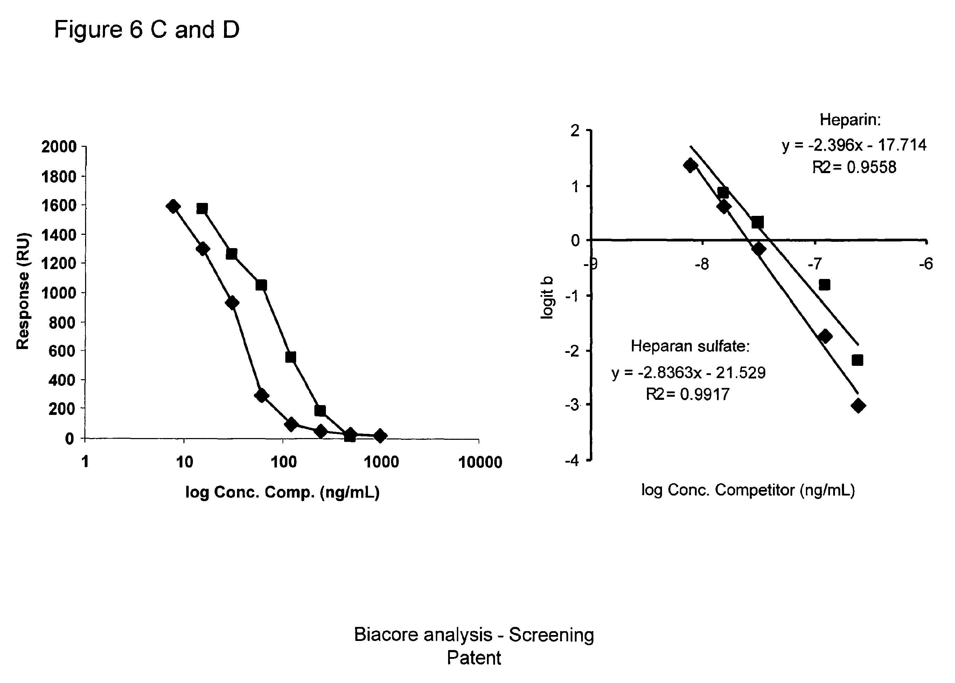

Figure 6 shows displacement of S100A9 binding to RAGE by soluble heparan sulfate and heparin. Sensorgrams for binding of 200 nM S100A9 to immobilized RAGE in the absence or presence of 7.81-1000 ng/mL heparan sulfate or 3.91-500 ng/mL heparin are shown in (A) and (B). Inhibition curves and log-logit plots are shown in (C) and (D) for heparan sulfate (◆) and heparin (■), respectively. IC₅₀ values for heparan sulfate and heparin were calculated to 25.7 and 40.4 ng/mL calculated after linear regression (equations are inserted). The value for heparin corresponds to ~ 8 × 10⁻⁹ M using the average molecular weight reported (4,000-6,000 Da). The molecular weight for heparan sulfate is not known. The binding of heparan sulfate and heparin to the RAGE surface is negligible (data not shown).

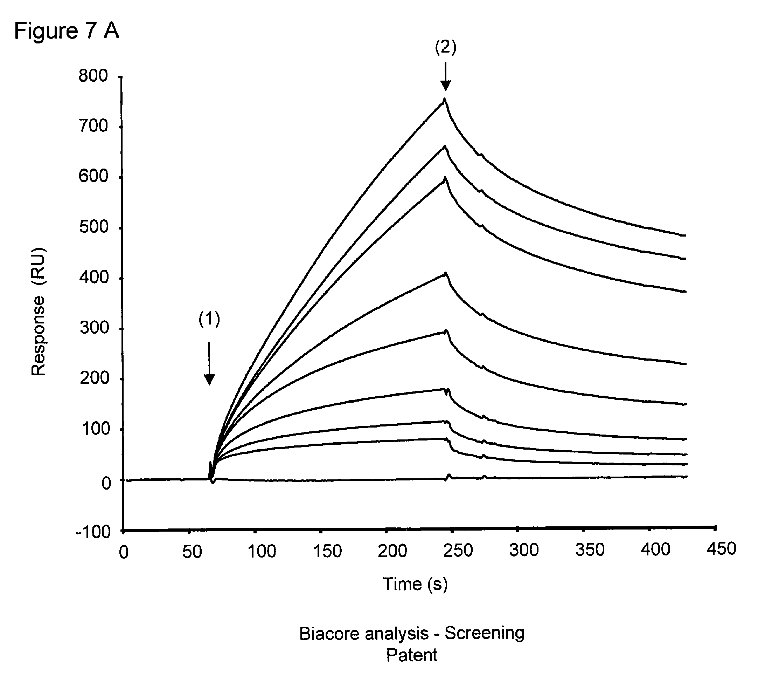

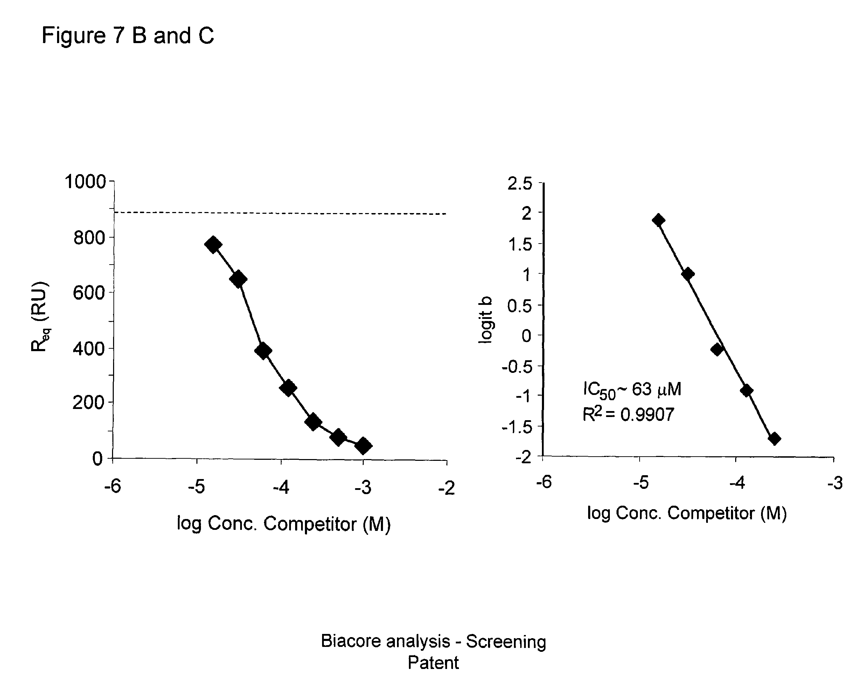

Figure 7 shows displacement of S100A9 binding to immobilized RAGE by soluble compound A. In (A), sensorgrams after injection of S100A9, 200 nM, in the absence or presence of compound A as competitor. Sensorgrams from top to bottom represent: S100A9 without and with 15.625, 31.25,62.5, 125, 250, 500 and 1000 µM compound A as competitor and from sample buffer without S100A9 and competitor. In (B), binding data is plotted as an inhibition curve (◆) and the response for S100A9 in the absence of competitor (dotted line). In (C), binding data is plotted after log-logit transformation. IC₅₀ values for compound A was calculated to 63 µM (r² 0.991). Arrows in (A) indicate start of the association phase (1), i.e. injection of S100A9 ± competitor, and dissociation phase (2), where running buffer is pumped over the surface. Injection time, flow rate and regeneration conditions were as in Figure 1.

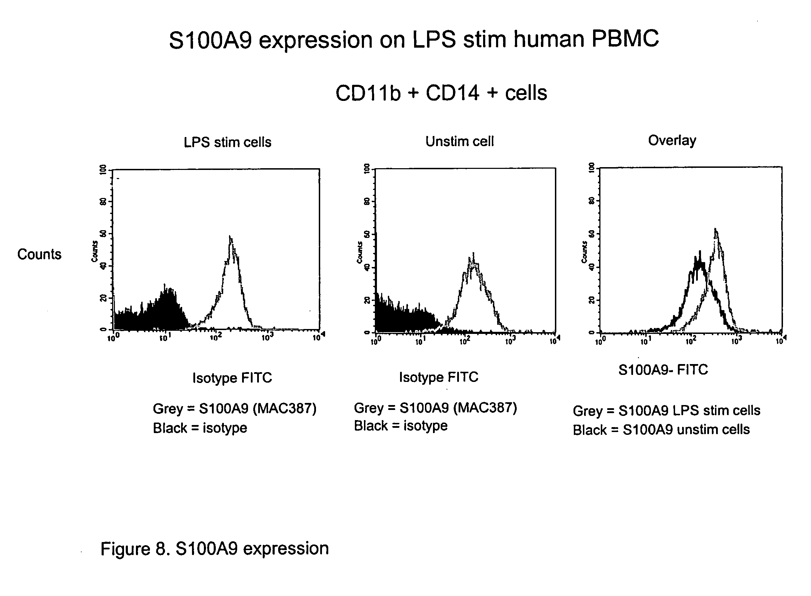

Figure 8 concerns S100A9 expression on LPS stimulated human PBMC. Human monocytes express S100A9 on the cell surface and the expression is increased by TLR4 (LPS) stimulation. The cells were gated for CD11b⁺CD14⁺ cells. S 100A9-specific staining of cells incubated either in medium alone (dark/blue) or together with LPS (lighter colour/green) are displayed.

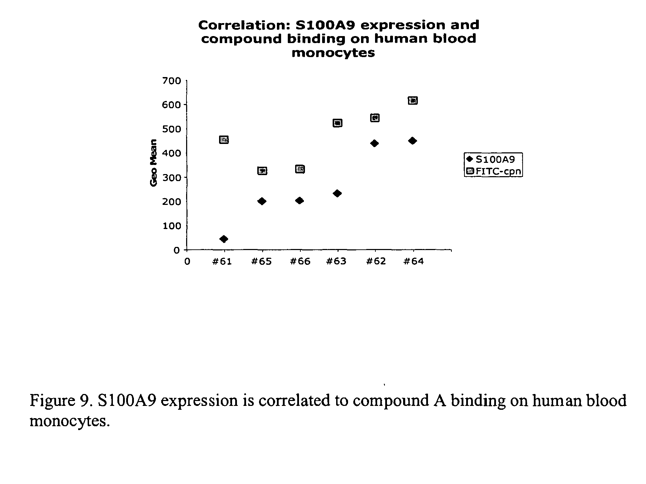

Figure 9 shows that S100A9 expression is correlated to compound A binding on human blood monocytes. Correlation between S 100A9 surface staining and binding of a fluorescent S100A9-binding compound to human blood monocytes PBL from 6 healthy volunteers were stained with fluorescence labelled S100-binding compound (diamonds) and fluorescent anti-S100A9 antibody (squares) and analysed by FACS after gating on the monocyte population.

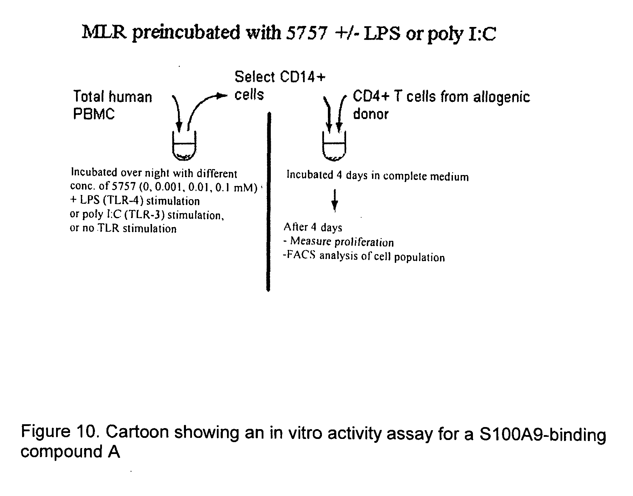

Figure 10 concerns a MLR preincubated with compound A. The cartoon shows an in vitro activity assay for a S100A9-binding compound A. Human PBL were purified using standard procedures and incubated for 24 hour in medium alone or in the presence of a TLR3 ligand (poly dI:dC, 20µg/ml) or a TLR4 ligand (LPS, 0.2µg/ml). Compound A was added to the various cultures at the indicated concentrations. After 24 hours the monocytes were purified using anti-CD14-conjugated magnetic beads and used as stimulators in an MLR reaction. Four days after the initiation of the cultures the cell populations were analysed using FACS.

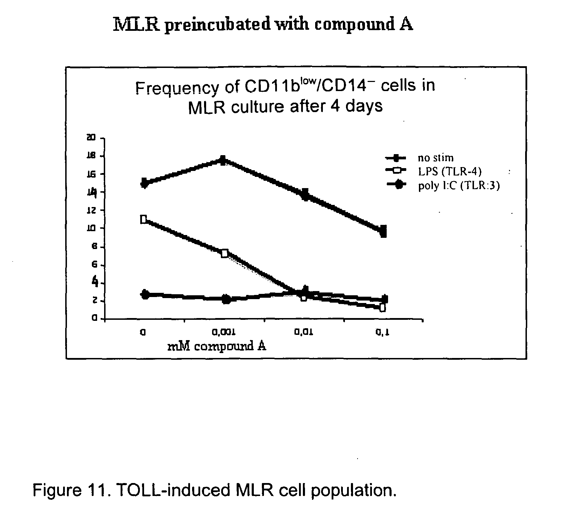

Figure 11 concerns a TOLL-induced MLR cell population. A specific, TOLL-induced MLR cell population is selectively diminished by the addition of S100A9-binding compounds to the pre-cultures. The frequency of a defined CD11^{blow}CD14⁻ cell population that emerges in MLR cultures only when TOLL stimulated CD14⁺ cells have been used as stimulators is dose-dependently reduced when S100A9 binding compound has been added to the pre-cultures.

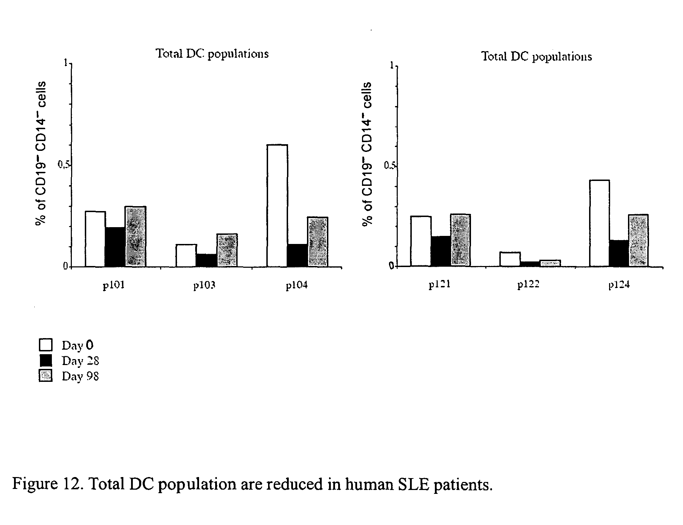

Figure 12 shows total DC population are reduced in human SLE patients. Total DC populations are reduced in human SLE patients treated with S100A9 binding compound. SLE patients treated with 1.5 mg/day (left panel) or 3 mg/day (right panel) have a reversible downregulation of total blood DC as determined by FACS analysis. Day 0 represents base-line values before dosing; day 28 the patients are at steady-state and day 98 the patients have been withdrawn from treatment for 14 days.

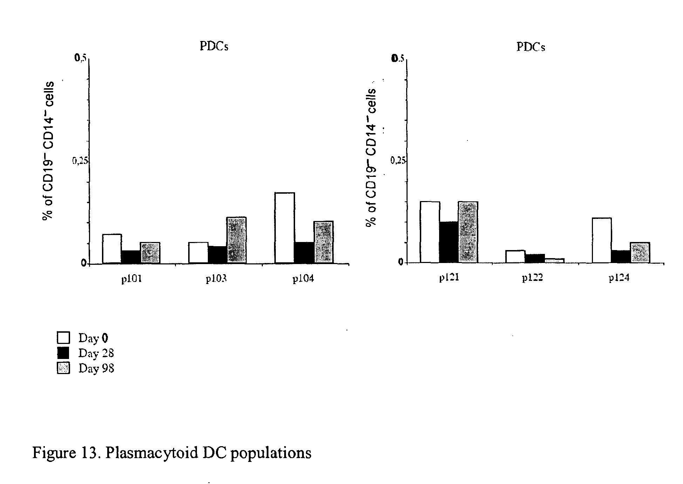

Figure 13 shows plasmacytoid DC populations are reduced in human SLE patients treated with S100A9 binding compound. SLE patients treated with 1.5 mg/day (left panel) or 3 mg/day (right panel) have a reversible downregulation of blood plasmacytoid DC as determined by FACS analysis. Day 0 represents base-line values before dosing, day 28 the patients are at steady-state and day 98 the patients have been withdrawn from treatment for 14 days.

### Description of the sequences mentioned herein

SEQ ID NO: 1 shows the sequence of S100A9, SEQ ID NO: 2 shows the sequence of RAGE and SEQ ID NO's 3 to 15 show the sequences of TLR's 1 to 13.

### Detailed description of the invention

The invention relates to a method for identifying compounds for use in therapy, in particular compound for use in cancer therapy or immunotherapy. The compounds may be for use in therapy of autoimmune disease or an inflammatory condition. Typically the compounds may be used to treat an individual that has, or is at risk of having, a condition that can be prevented or treated by modulation of the immune system. The condition may be an autoimmune disease, such as type I diabetes, systemic lupus erythematosus, rheumatoid arthritis or multiple sclerosis. In one embodiment the condition is cancer. The individual to be treated in the therapy is typically a mammal, and preferably a human. Further the inventors have carried out the binding assays in conditions under which only S100A9 binds ligand strongly, and other S100 proteins do not, and thus preferred methods of the invention are based on binding assays carried out under such conditions.

The method of the invention identifies therapeutic compounds based on their ability to modulate the interaction between S100A9 and a ligand of S100A9, or on their ability to bind S100A9 in a manner which would disrupt binding of S100A9 with a ligand. The ligand is typically RAGE or a TLR (Toll like receptor). The TLR is preferably TLR4, but may be any TLR which binds S100A9, such as TLR1, TLR2, TLR3, TLR5, TLR6, TLR7, TLR8, TLR9, TLR10, TLR11, TLR12 or TLR13.

The method may be based on any suitable assay. The assay may measure or detect any of the physical properties mentioned herein, and a compound may be selected based on any such physical property. As can be seen in the Examples the inventors have characterized the properties of a therapeutically effective compound (termed compound A in the Examples), and thus the assay may determine whether a candidate compound has a relevant physical property which is the same or similar to the compound A.

Typically the method comprises determining whether a candidate compound is able to affect the binding of S100A9 and a ligand. The candidate compound may bind to S100A9 and cause a change in the conformation of S100A9, for example by inhibiting the binding of calcium or zinc to S100A9. Thus the assay may comprise providing a candidate compound to S100A9 and the ligand and determining whether the binding of S100A9 to the ligand has been affected. The assay may comprise allowing the candidate compound to bind S100A9 and then determining whether the binding would affect the binding of S100A9 to RAGE or a TLR, for example by testing whether the candidate compound binds S100A9 at the same site as RAGE or a TLR.

In a preferred embodiment the method comprises identifying a compound that modulates the interaction of S100A9 and a ligand. The term "modulate" as used herein refers to both upregulating (i.e. activation or stimulation, e.g. by agonizing or potentiating) and downregulation (i.e. inhibition or suppression, e.g. by antagonizing, decreasing or inhibiting) of an activity. The compound may be used to modulate the immune response in vivo.

In the method S100A9 and/or the ligand and/or the compound are preferably used in isolated form or substantially isolated form. Typically they are not present in or on the surface of cells and/or other proteins are not present. Typically less than 10, such as less than 5 or less than 3 other types of protein are present. Preferably calcium and zinc ions are present. In one embodiment 0.1 to 5 mM calcium ions, such as 0.5 to 2 mM calcium ions or about 1mM calcium ions and/or 1 to 100uM zinc ions, such as 5 to 50uM zinc ions or about 10uM zinc ions are present.

In the method S100A9 or the ligand may be immobilized to a solid surface, and preferably the ligand is immobilized. Immobilisation may be achieved by any suitable means, for example by amine coupling. The solid surface may be one which is arranged so that changes in its surface characteristics are detected by surface plasma resonance. The immobilized molecule should still be capable of binding to S100A9 (where a ligand is immobilized) or the ligand (where S100A9 is immobilized).

In the method the binding of S100A9 and ligand is typically measured over a certain time period, for example over 10 to 12000 or 20 to 6000 or 60 to 500 seconds, which may allow the kinetics of the binding to be observed, and thus allow compounds to be selected based on the kinetics of binding. In addition the method may comprise measurement of the effects of different amounts/concentrations of the compound, thus allowing selection of compounds which have a dose-dependent effect.

The method of the invention preferably measures and/or selects a compound based on one or more of the following characteristics:
- whether there is a 1:1 binding between the compound and S100A9 or ligand, for example by analysis of the effect of different concentrations of the compound,
- the IC50 of the compound, and preferably whether the IC50 is from 1 nM to 200uM, for example from 10nM to 150um, 0.5uM to 100uM or 20 to 70uM,
- the effect of the compound on the on-rate and/or off-rate of S100A9 and ligand, or
- the level of decrease of binding of S100A9 and ligand,
- whether the compound binds to S100A9 or ligand under physiological (in vivo) conditions,
- whether the binding is specific,
- whether the binding is reversible or irreversible, or
- whether the compound causes a change in the structure of S100A9 or ligand.

In one embodiment at least 100, such as at least 1000, or at least 10,000 candidate compounds are tested. At least 1, 10, 50, 100 or more or all of the compounds that are tested preferably have one or more of the following characteristics:
- they are not proteins or nucleic acids,
- they are not lipids,
- they are not toxic to mammals or humans,
- they are soluble in water,
- their structure is characterized or uncharacterized,
- they are from a library of compounds which may, for example, have been synthesized together,
- they are small organic molecules (typically containing carbon, hydrogen and generally also oxygen), and preferably having a relative molecular weight of at least 100, and less than 1000, such as a relative molecular weight of less than 600.

In one embodiment of the method less than 5%, for example less than 10% or less than 15% of the compounds which are tested are selected.

### Forms/variants of S100A9 or ligand that may be used in the method

Any suitable form of S100A9 or ligand may be used in the method. Thus they may used in the form of homologues and/or fragments of naturally occurring forms. The term "homologues and/or fragments" includes fragments of homologues of the relevant molecule. Homologues and/or fragments of any of the specific sequences disclosed herein may be used or of any naturally occurring isoform (for example of any mammalian species). Preferably a homologue and/or fragment of a human S100A9 or ligand is used. The homologue and/or fragment are termed "variant" in the discussion below.

The variant may be capable of complementing one or more activities of the naturally occurring molecule. Preferably the variant is capable of binding to S100A9 (if it is a variant of the ligand) or to the ligand (if it is a variant of S100A9). The variant polypeptide may comprise sequence which is homologous to all or part of S100A9 or the ligand. Thus the variant may be a fusion protein. The variant thus generally comprises at least the binding site of S100A9 or the ligand.

In a preferred embodiment the variant comprises the extracellular domain of the ligand. In the case where the variant is a variant of RAGE preferably at least 1, 2 or 3 of the immunoglobulin domains (or homologues of the domains) of RAGE are present in the variant. A preferred RAGE variant is described in the Examples and comprises amino acids 1 to 344 of human RAGE fused to the Fc region of human IgG1. In the case where the variant is a variant of S100A9 preferably at least the N-terminus helix 4 and linker region (or homologues of helix 4 and/or the linker region) are present in the variant and/or preferably at least the two histidine amino acids of helix 4 are present in the variant (which are important in binding zinc) and/or preferably the five histidines in the C terminus which are important in zinc coordination/binding are present in the variant and/or preferably the variant is capable of binding zinc.

In one embodiment the form of S100A9 or ligand which is used binds an antibody that binds the naturally occurring form. In the case of RAGE the form which is used may bind to MAB11451 that is available from R & D Systems.

The variant is typically at least 100 amino acids long, such as at least 200 amino acids long, up to, for example, 2000 amino acid in length.

The forms of S100A9 and/or ligand may be modified, e.g. post-translationally modified. For example, they may be glycosylated or comprise modified amino acid residues. The ligand is preferably glycosylated, for example in a glycosylated form which is obtainable by expression in a mammalian (preferably human) cell.

In general use of other S100 naturally occurring proteins apart from S100A9 is not included in the method of the invention.

Any suitable monomer/dimer/multimer form of S100A9 or ligand may be used. S100A9 may be used in monomer, dimer or multimer form. It is preferably used in dimer form (i.e. comprising two S100A9 proteins). The ligand may be used in monomer or dimer form. RAGE is preferably used in dimer form (comprising two RAGE proteins).

Expressed S100A9 proteins or ligands (including fragments and homologues) can be recovered and purified from recombinant cell cultures by methods including ammonium sulfate or ethanol precipitation, acid extraction, anion or cation exchange chromatography, size-exclusion chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

Any polypeptides mentioned herein may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be post-translationally modified and in particular they may be glycosylated or may be non-glycosylated.

### Homologues

Homologues of sequences are referred to herein. Such homologues typically have at least 20% homology, preferably at least 30%, 40%, 50%, 60%, 70%, 80%, 90%, 95%, 97% or at least 99% homology, for example over a region of at least 15, 20, 30, 100 more contiguous amino acids or over the length of the entire molecule. The homology may be calculated on the basis of amino acid identity (sometimes referred to as "hard homology").

For example the UWGCG Package provides the BESTFIT program which can be used to calculate homology (for example used on its default settings) (Devereux et al (1984) Nucleic Acids Research 12, p387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologous sequence typically differs by less than 5, 10, 20 or 50 mutations (which may be substitutions, deletions or insertions of nucleotide or amino acids). These mutation may be measured across any of the regions mentioned above in relation to calculating homology. The substitutions are preferably conservative substitutions. These are defined according to the following Table. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

### Further testing of the identified compound

A compound identified by the above method (which is non-cellular and not in vivo) may be further tested to determine whether it modulates the immune response, and preferably whether it decreases the immune response. Compounds may be selected if they are found to modulate the immune response. Any suitable cell based assay or animal model assay may be used. Preferably the assay will comprise monocytes which express S100A9, and will test whether the compound is able to modulate the growth or activity of such monocytes. The assay may determine whether the compound modulates S100A9 expression on the monocytes. The assay may determine whether the compound binds to a CD11b+CD14+ population of cells and/or whether the compound modulates (for example decreases) the level or activity of such cells.

Thus the compound may be contacted with a monocyte (such as a CD11b+CD14+ monocyte) to determine whether the compound modulates the growth or activity of the moncyte.

The assay may be in the form of a mixed lymphocyte reaction (MLR), for example one comprising monocytes which have been stimulated with TOLL.

The compound may be tested to determine whether it modulates (for example increases or decreases the numbers of) monocytes (for example dendritic cells) in an animal model and/or for therapeutic efficacy in an animal model. The animal model may be of any of the conditions mentioned herein, such as cancer, autoimmune disease or an inflammatory condition. The animal is preferably a rodent, such as a mouse or rat.

Thus the compound may be administered to an animal and a sample of containing monocytes may be taken from the animal to determine whether the compound causes a decrease in the number or activity of monocytes in the animal. In another embodiment the compound may be administered to an animal with a cancer or autoimmune disease, or an animal at risk of having cancer or an autoimmune disease, and whether or not the compound treats or prevents the autoimmune disease is determined.

### Administration

Compounds identified by the method of the invention may be formulated into pharmaceutical composition. The pharmaceutical composition may be used in clinical treatment of diseases resulting from autoimmunity such as multiple sclerosis, insulin-dependent diabetes mellitus, systemic lupus erythematosus, rheumatoid arthritis, inflammatory bowel disease and psoriasis and, furthermore, diseases where pathologic inflammation plays a major role, such as asthma, atherosclerosis, stroke and Alzheimer's disease. More particularly, it may be used for the treatment of, for example, multiple sclerosis and its manifestations. The pharmaceutical composition may be used in the treatment of cancer.

The formulation will depend upon factors such as the nature of the substance and the condition to be treated. Any such substance may be administered in a variety of dosage forms. It may be administered orally (e.g. as tablets, troches, lozenges, aqueous or oily suspensions, dispersible powders or granules), parenterally, subcutaneously, intravenously, intramuscularly, intrasternally, transdermally or by infusion techniques. The substance may also be administered as suppositories. A physician will be able to determine the required route of administration for each particular patient.

Typically the substance is formulated for use with a pharmaceutically acceptable carrier or diluent. The pharmaceutical carrier or diluent may be, for example, an isotonic solution. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for intravenous or infusions may contain as carrier, for example, sterile water or preferably they may be in the form of sterile, aqueous, isotonic saline solutions.

A therapeutically effective non-toxic amount of substance is administered, typically to a patient (preferably a human patient) in need thereof. The dose may be determined according to various parameters, especially according to the substance used; the age, weight and condition of the patient to be treated; the route of administration; and the required regimen. Again, a physician will be able to determine the required route of administration and dosage for any particular patient. A typical daily dose is from about 0.001 to 50 mg per kg, preferably from about 0.1mg/kg to 10mg/kg of body weight, according to the activity of the specific inhibitor, the age, weight and conditions of the subject to be treated, the type and severity of the disease and the frequency and route of administration. Preferably, daily dosage levels are from 0.5 mg to 2 g.

The following Examples illustrate the invention:

### Examples

### Proteomic Screen

When investigating compounds that bind S100A9 or RAGE in a proteomics screen, no compounds were identified which bound RAGE, whilst compounds which bound S100A9 were identified. Thus in order to identify compounds that modulate the interaction of S100A9 with a ligand such as RAGE, it is more efficient to look for compounds that bind to S100A9.

Notably, a distinguishing feature of S100A9 is its extended C-terminus, rendering it considerably larger than other S100 family members. Furthermore, S100A9 contains two EF-hands motifs (helix-loop-helix motif) flanked by conserved hydrophobic residues and separated by a hinge region. S100A9 shares this theme with the S100 protein family. The sequences of the hinge region and the C-terminal helix, H4, are the most variable among the S100 proteins. Binding of the S100A9 protein, and the S100A9 and S100A8 complex, to endothelial cells, requires the presence of calcium and zinc ions.

### The Biacore Assay

The Surface Plasmon Resonance (SPR) technology, such as the BIAcore assay system, was chosen as a first step of the screening method. The assay format provides affinity and kinetic information on the interaction of a ligand and an immobilized receptor. Moreover, is the assay format particular useful in mimicking physiological conditions without labeling any of the interactants. Especially, where avidity and multivalent presentations are involved, and the monomeric interaction is weak, is the chosen assay format useful. Compounds may then be tested for their ability to modulate the ligand - receptor interaction in an inhibition assay where the ligand is co-injected with varying concentrations of compound to determine whether the compound inhibits this interaction in a dose-dependent manner. Finally, compounds may be compared as to their inhibitory potency.

### The immobilization of RAGE to a chip

The RAGE receptor was immobilized onto a biosensor surface using random amine coupling chemistry to a CM5 chip in a Biacore assay system. The applied form of RAGE, is a recombinant human RAGE receptor, where the extracellular domain of RAGE, amino acid 1-344, is fused to the Fc region of human IgG₁. The chimeric protein was expressed in the mouse myleoma cell line, NSO, as a disulfide-linked homo-dimeric protein with a calculated molecular mass of 61 kDa. Human RAGE possesses two conserved glycosylation sites and heterogeneity in glycosylation results in subpopulations of RAGE, differing in *N*-carboxylated glycans displaying different ligand binding properties, (Srikrishna G, et al., J Immunol 175, 5412-5422, 2005). Due to extensive glycosylation of RAGE relative to that of the Fc domain and a higher mass of the latter, it was also assumed that chimeric RAGE/Fc will be coupled predominantly via the Fc domain and result in a semi-oriented coupling to the sensor chip. Thus, the chosen assay format allows the extracellular part of RAGE to be exposed to ligand binding, and reconstituting a biological model where anchored membrane receptor interacts with soluble ligands.

The biological activity of immobilized RAGE was tested by injecting a specific anti-human RAGE monoclonal antibody recognizing the extracellular domain of human RAGE, over the surface of the immobilized RAGE. Figure 2, shows sensograms for binding of 12.5 - 200 nM anti-RAGE over immobilized RAGE. An apparent dissociation constant, K_{D}, in the nanamolar range was obtained.

### The novel interaction S100A9 and RAGE

Binding of S100A9 to RAGE was demonstrated after the immobilization of the rhRAGE-FC by random amine coupling to a chip. In Figure 2, sensograms from injection of 37.5-200 nM S100A9 over immobilized RAGE are shown. The responses are plotted versus concentration of S100A9, a sigmoidal dose-response curve is obtained although saturation is not reached within the concentration range used, Figure 2B. The S100 proteins are calcium-binding proteins and some also require low concentrations of Zn²⁺ to adapt a biologically active conformation. Titration of Zn²⁺ for optimal binding of 100 nM S100A9 to RAGE was performed in presence of Ca²⁺. The optimal zinc ion concentration was determined to 10 µM Zn²⁺ in the presence of calcium, Figure 3.

Under the experimental conditions used, S100A9 showed more than 10-fold higher binding to RAGE than the next group of RAGE binders (AnxII, S100A12, and S100A8/9), Figure 5. In vivo, S100A9 usually forms hetero-dimeric complexes with S100A8.

Interestingly, the significantly lower binding of the S100A8/9 hetero-dimer to RAGE than for homo-dimeric S100A9 implicates that binding to RAGE occurs via S100A9 and that the binding site is partially blocked in the S100A8/9 hetero-dimer. The finding that weak or no binding was obtained with putative RAGE ligands like S100A1, S100A12, S100B and S100P indicates that S100A9, unique among the S100 members to interact strongly with glycosaminoglycans and carboxylated glycans, may interact with glycans on RAGE. The S100A9 protein binding to endothelial cells has been shown to involve glycosaminoglycans structures and may be blocked by heparin and heparin sulfate. Displacement of S100A9 binding to RAGE by soluble heparin or heparan sulfate was demonstrated for the immobilized RAGE to a chip, Figure 6. The finding demonstrates the biological relevance of the disclosed interaction between S100A9 and RAGE.

### The compounds are modulators of the S100A9 to RAGE interaction

The binding of S100A9 to immobilized RAGE is modulated by compound A, Figure 7. Dose-dependent inhibition is obtained with an IC₅₀ value of 63 µM for the compound A.

### The TLR4 immobilized on chip

The TOLL-like receptor 4 was immobilized on the sensor chip. Binding to S100A9 was detected.

### In vitro assay - MLR

### S100A9 surface expression of human monocytes

The S100A9 molecule is present on the cell surface of only a minority of human PBMC. This can be illustrated by staining total human PBMC with a flourescien-labelled antibody specific for S100A9 followed by analysis in a FACS analyser. As shown in Figure 8, only a small population of the cells bound the antibody while the majority of the cells had a flourescence equal to that seen after incubation with an isotype matched antibody with unknown specificity as a negative control. Furthermore, when the cells had been pre-incubated with a TLR4 ligand (LPS) for 24 hours the fraction of PBMCs that expressed S100A9 remained more or less constant while the staining intensity of the population was increased, indicating that each S100A9-binding cell expressed more molecules on the cell surface.

Monocytes have been described as one of the S100A9 expressing cell types in human blood (Zwadlo et al. (1988) Clin.Exp.Immunol. 72, 510). Indeed, essentially all monocytes can be shown to express S100A9 on the cell surface by FACS analysis (Figure 9) and also on this cell population the level of surface expression is increased after stimulation with a TLR ligand.

Knowing that TOLL stimulation increased the surface expression of S100A9 on human monocytes in vitro it was of interest to address whether blockade of S100A9 could have any functional consequence on human monocytes. To this end we designed an in vitro assay, Figure 10.

Figure 11 shows an example of such analysis. We have noted that in the MLR reactions using TOLL-stimulated monocytes as stimulators cells, a specific cell population could be detected after 4 days. This cell population was CD11b^{low} and CD14⁻ as determined by FACS analysis. If the stimulator cells were pre-incubated in medium only, this population did not appear (Figure 11). Addition of a S100A9 binding compound to the preincubation cultures at the indicated concentrations reduced the percentage of CD11b^{low}CD14⁻ cells in the MLR reaction in a dose-dependent fashion. This was true independently of whether the pre-incubation cultures had been stimulated with a TLR3 or a TLR4 ligand. Hence, this simple in vitro assay can be used to functionally assess S100A9 binding compounds influence on biological function in vitro.

The MLR assay is described in Bach & Hirschhorn (1964), Science 143, 813.

### In vitro assay for S100A9 binding compounds modulating TOLL-dependent stimulation of human PBMC

Human PBMC were incubated over night in medium only or together with a TLR4 ligand (LPS) or a TLR3 ligand (polidI:dC). After this pre-incubation period , monocytes were purified based on their CD14 expression using magnetic beads coated with anti-CD14 antibodies. These CD14 cells were in turn used as stimulators in a Mixed Lymphocyte Reaction (MLR) with PBMC from an allogeneic donor. After 4 days the MLR reaction can be analysed using various methods.

### Treatment with S100A9 binding compounds has effects on dendritic cell populations in human blood.

Human monocytes belong to a cell type that is usually referred to as Antigen Presenting Cells (APC). The monocytes can, upon proper stimulation, exit the blood stream into tissues and coupled to this process is their differentiation into distinct cellular phenotypes such as macrophages and dendritic cells (DC). DC are also found circulating in the blood stream and can be further subdivided based on surface markers. DC are also regarded as the major immunoregulatory type of APC. It was therefore of interest to investigate whether treatment of human subjects with S100A9 binding compound influenced the levels of DC in the blood. We here took advantage of an ongoing Phase Ib study where SLE patients are treated with compound A, a S100A9 binding compound. Blood samples from the patients were taken before treatment (Day 0), at steady-state one month after treatment (day 28) and two weeks after treatment was stopped (Day 98). Two dose group of three patients each were available for analysis; 1.5 mg/day and 3 mg/day. PBMCs were analysed at these times for the presence of totals DC (Figure 12) and plasmacytoid DC (Figure 13). As can be seen in Figures 15 and 16, both the percentage of total DC and of plasmacytoid DC were reduced when the patients were under treatment with compound A compared to pre- and post-treatment levels. Thus, treatment of humans with S100A9 binding compounds has an effect on APC in vivo.

### MATERIALS AND METHODS

### Compound A

The compound A was synthesized according to the methods disclosed in US 6,077,851. Compound A is described in formula (I) of Figure 1 of the document.

### Biosensor analysis

Binding analysis was performed using the "surface plasmon resonance" (SPR) technology with a Biacore 3000™ system. This technology allows analysis of an interaction between proteins and small molecules in a real-time and label-free manner. A protein or low-molecular weight compound is injected over a surface (a CM5 sensor chip) with a covalently coupled ligand and the signal produced, expressed as resonance units (RU), is directly proportional to the number and mass of the molecule interacting with the ligand on the surface. Immobilization, regeneration scouting, surface performance tests and binding studies were conducted basically according to the instructions by the manufacturer. Kinetic evaluation of binding data was carried using the BIAevaluation Software Version 3.2 (Biacore AB). Optimization of conditions for immobilization and binding analysis is carried out for each interaction to be studied. A biosensor analysis cycle consists of: (i) injection of S100A9 over the RAGE surface for 3 min (association phase) or (i) injection of S100A9 over the TLR4/MD-2 surfaces, in the absence or presence of 100 ng/mL LPS, for 3 min (association phase); (ii) injection of running buffer for 2.5 min (dissociation phase); (iii) injection of a 30 s pulse of 10 mM glycine-HCl, pH 2.0-2.125 (regeneration phase); and (iv) injection of running buffer for 2 min (stabilization after regeneration) at a flow rate of 30 µl/min.

Identity and homogeneity of S100 proteins were tested on SDS-PAGE.

### Immobilization of RAGE

Recombinant human RAGE was obtained from R&D Systems (cat. No. 1145-RG) as a fusion protein of the extracellular domain of RAGE, comprising a.a. 1-344, and the Fc region of human IgG₁ via a peptide linker. The chimeric protein was expressed in the mouse myleoma cell line NSO as a disulfide-linked homo-dimeric protein with a calculated molecular mass of 61 kDa. Prior to covalent coupling to the sensor chip, buffer was changed to a standard Biacore buffer (10 mM Hepes, 0.15 M NaCl, pH 7.4, containing 0.005% v/v Surfactant P20; HBS-P) using Fast Protein Desalting Micro-Spin Columns (Pierce). RAGE was diluted in an immobilization buffer shown to give sufficient pre-concentration and then covalently coupled using the "Aim at immobilization level" function in the Biacore Wizard to obtain various coupling densities. Activity of immobilized RAGE was tested by injecting a specific anti-human RAGE monoclonal antibody over the surface. Due to extensive glycosylation of RAGE relative to that of the Fc domain and a higher mass of the latter, it was also assumed that chimeric RAGE/Fc will be coupled predominantly via the Fc domain and result in a semi-oriented coupling to the sensor chip. HBS-P, with a final concentration of 1 mM CaCl₂ and 10 uM ZnCl₂, was used as sample and running buffer throughout the study. For regeneration of the surface after each cycle, a pulse of 10 mM glycine-HCl, pH 2.125, was used. To determine optimal zinc concentration for binding of S100A9 protein to RAGE, the interaction was allowed to take place at a fixed calcium concentration (1 mM) and with the zinc concentration titrated from zero to ten µM.

### Interaction of soluble S100A9 with the RAGE surface

Recombinant S100A9, expressed as the full-length sequence of native protein in *E. coli* was obtained using a four-step purification protocol. Purity was checked on over-loaded SDS-PAGE gels and found to be > 95% homogeneous. Buffer was changed to HBS-P using the Fast Protein Desalting Spin Columns and concentration determined in the BCA protein assay from Pierce with bovine albumin as standard. A 10 µM stock solution based on the homodimeric molecular weight of S100A9 was prepared and stored in aliquots frozen at -25°C. From this, a working solution of 1000 nM in HBS-P was prepared and diluted to the concentration to be tested with ZnCl₂ and CaCl₂ added to a final concentration of 10 µM Zn²⁺ and 250 or 1000 µM Ca²⁺ according to the buffer system proposed by Robinson et al. (2002).

### Immobilization of TLR4/MD-2 complex

Recombinant human TLR4/MD-2 was obtained from R&D Systems (cat. no. 3146-TN/CF) as a non-covalently associated protein of the respective extracellular domains or as a fusion protein between the extracellular domains of murine TLR4 and MD-2, 'LPS-Trap' (Brandl K, et al. J Endotox Res 11, 197-2006, 2005). Prior to covalent coupling to the sensor chip, buffer was changed to a standard Biacore buffer (10 mM Hepes, 0.15 M NaCl, pH 7.4, containing 0.005% v/v Surfactant P20; HBS-P) using Fast Protein Desalting Micro-Spin Columns (Pierce).

### Interaction of soluble S100A9 with the TLFR4/MD-2 surface

Recombinant S100A9, expressed as the full-length sequence of native protein in *E. coli* was obtained using a four-step purification protocol. Purity was checked on over-loaded SDS-PAGE gels and found to be > 95% homogeneous. Buffer was changed to HBS-P using the Fast Protein Desalting Spin Columns and concentration determined in the BCA protein assay from Pierce with bovine albumin as standard. A 10 µM stock solution based on the homodimeric molecular weight of S100A9 was prepared and stored in aliquots frozen at -25°C. From this, a working solution of 1000 nM in HBS-P was prepared and diluted to the concentration to be tested with ZnCl₂ and CaCl₂ added to a final concentration of 10 µM Zn²⁺ and 250 or 1000 µM Ca²⁺ according to the buffer system proposed by Robinson et al. (2002).

### Competition assay

In order to analyze compounds as potential inhibitors of the S100A9 - RAGE interaction, S100A9 was injected over the RAGE surface in the absence or presence of serially diluted compound. Compounds were dissolved either directly as a 2 mM solution in HBS-P or in double-distilled water at 5 or 10 mM concentration and stored in aliquots at -25°C. In the latter case, compounds were diluted to a 2 mM working solution by adding 10 % v/v 100 mM Hepes, 1.5 M NaCl, pH 7.4, 0.05 v/v Surfactant P20. Biomolecules known to interact with S100A9 in a calcium-dependent manner and with an affinity in the low nanomolar range are glycosaminoglycans like heparin and heparan sulfate (Robinson et al., 2002). Therefore, heparin and heparan sulfate were used as positive controls in the assay to test their ability to inhibit the S100A9 interaction with immobilized RAGE.

### Evaluation of binding data

Binding data was evaluated by fitting to standard binding models, where appropriate, or calculation of response at late association phase using Steady state affinity function using the BIA evaluation Software version 3.2. Affinity was determined either from kinetic analysis (on- and off-rates) or from plotting of responses versus concentration of S100A9 in a saturation curve. In the inhibition assay format, the concentration of competitor yielding 50 % inhibition of S100A9 bound in the absence of competitor (IC₅₀ value) was calculated by fitting binding data to a one-site competition model in GraphPad Prism or after log-logit transformation of data and linear regression.

### Purification of splenic DCs and human PBMCs

Spleens and lymph nodes fom treated and untreated mice were cut into small pieces and treated with collagenase and DNA:ase to release the DCs from the tissue. The DCs were positively selected using CD11c-magnetic microbeads. Human PBMCs were isolated from whole blood using gradient centrifugation.

### MLR reactions

Human PBMCs were cultured in vitro over night in tissue culture medium. The cells were either activated by the TLR ligands LPS or poly I-C or not activated. Various concentrations of compound A was added to parallel cultures. After the over night culture CD14-positive cells were purified using CD14-magnetic beads. These cells were γ-irradiated and used as stimulator cells in the MLR-reaction. From another donor, CD4-positive cells were purified using CD4-magnetic beads. The proliferation of the responder T cells was determined on day 4 of culture by measuring incorporation of ³H-thymidine.

### Analysis of cell subsets by flow cytometry

Collagenase/DNAse-treated spleen or lymph node cell suspensions or purified DCs were blocked with anti-Fc mAb and stained with the following fluorescently labeled antibodies: CD11c, CD4 and CD8. Human PBMCs or cells from MLR-reactions were stained with the following fluorescently labeled antibodies: CD11b, CD14 and S100A8/A9. Stained cells were analysed by flow cytometry. Dead cells were excluded using 7-actinomycin D

### Autoimmune disease models

Compound A shows a dose dependent inhibition of disease induction in several animal models of autoimmune disease. Compound A is suitable for use in the clinical treatment of diseases resulting from pathologic inflammation and autoimmunity, e.g., systemic lupus erythematosus (SLE), insulin-dependent diabetes mellitus (IDDM), collagen induced arthritis (CIA), multiple sclerosis (MS) and rheumatoid arthritis (RA). Compound A inhibits disease in a spontaneous animal model for systemic lupus erythematosus (SLE) in MRL *lpr*/*lpr* mice, in a spontaneous model for type I diabetes in nonobese diabetic (NOD) mice and in an induced model for multiple sclerosis, i.e., acute experimental autoimmune encephalomyelitis (aEAE) induced by immunization with spinal cord homogenate (Table 1). All these models have been the most widely used models for the human diseases.

### Experimental Lupus Model in MRL lpr/lpr Mice

The MRL *lpr*/*lpr* mouse spontaneously develops an autoimmune disease resembling human SLE. The SLE-like disease in these mice is characterized by immune complex-mediated glomerulonephritis measured as proteinuria and hematuria, enlargement of spleen and lymph nodes and production of anti-double stranded DNA (dsDNA) antibodies. Clinical signs can be detected at approximately 8 weeks of age. The main cause of death in these mice is renal failure. The mice were used for experiments from the age of 14-15 weeks. The development of glomerulonephritis measured as proteinuria and hematuria was followed. The mice were sacrificed after development of full SLE-like disease. Percent survival was calculated at the end of the experiment when the mice had reached the age of 23-24 weeks. Compound A (1 or 5 mg/kg/day) was administered in drinking water from 14-15 weeks and throughout the experiment.

### The nonobese diabetic (NOD) mouse model for autoimmune insulin-dependent diabetes mellitus (IDDM)

The NOD mouse spontaneously develops autoimmune IDDM and serves as an animal model for the human type I diabetes. NOD mice start to develop clinical symptoms of diabetes at 14-16 weeks of age. Mice were used in experiments from the age of 10 weeks. The development of overt diabetes was evaluated by measuring the existence of glucose in urine. If positive for glucose in urine, the animals were scored diabetic and sacrificed. Treatment with compound A (0.008, 0.04, 0.2, 1 or 5 mg/kg/day) was administered in the drinking water from 10 weeks to 26 weeks of age. Treatment with compound A (1 or 5 mg/kg/day) was administered in the drinking water from 5 weeks to 12 weeks of age.

### Acute Experimental Autoimmune Encephalomyelitis (aEAE) Model

EAE is a murine model for autoimmune inflammatory disease of the central nervous system. EAE in the mouse shares many features with the human disease multiple sclerosis. SJL/N female mice were used for the experiments. Complete Freund's adjuvant containing H37Ra mycobacteria was emulsified with an equal volume of mouse spinal cord homogenate (MSCH). The inoculum was injected intradermally at the base of the base of the tail. Pertussis toxin was injected i.p. at day 0 and 3 after immunization. The disability symptoms appear about eight days post immunization (p.i.) and peaks around 12 days p.i. Treatment with compound A (0.04, 0.2 or 1 mg/kg/day) was given at days 3 to 7 and 10 to 12 p.i. Control animals received saline. The animals were scored for clinical signs of paralytic disease on a scale from 0 to 5 in the following way; 0, normal; 1, limp tail; 2, hind limb paresis; 3, hind limb paralysis, and limp foreleg; 4, bilateral hind and fore limb paralysis; 5 death. Clinical scores were monitored daily from day 9 until the end of the experiment at day 14. Treatment effects were calculated as percent inhibition of mean clinical scores compared to saline treated controls.

**Table 1**

| **Survival (%), MRL *lpr*/*lpr* model of SLE** | | | |
|---|---|---|---|
| **Treatment** | **Number of mice - start** | **Number of mice - end** | **% Survival at 22-23 weeks of age** |
| Control | 7 | 1 | 14% |
| Compound A (1 mg/kg/day) | 7 | 6 | 86% |
| Compound A (5 mg/kg/day) | 5 | 5 | 100% |

| **Diabetic mice (%), NOD model of IDDM** | | | |
|---|---|---|---|
| **Treatment** | **Dose (mg/kg/day)** | **% Diabetic Mice at 26 weeks of age** | |
| Control | - | 80% | |
| Compound A | 0.008 | 70% | |
| Compound A | 0.04 | 50% | |
| Compound A | 0.2 | 15% | |
| Compound A | 1 | 0% | |
| Compound A | 5 | 0% | |

| **Disease inhibition(%), aEAE model in SJL mice** | | | |
|---|---|---|---|
| **Treatment** | **Dose (mg/kg/day)** | **Mean % inhibition** | |
| Compound A | 0.04 | 46 | |
| Compound A | 0.20 | 75 | |
| Compound A | 1 | 98 | |

SEQUENCE LISTING

<110> Active Biotech AB

<120> SCREENING METHOD

<130> N.101332

<140> 07251762.6
<141> 2007-04-26

<160> 15

<170> PatentIn version 3.4

<210> 1
<211> 114
<212> PRT
<213> Homo sapiens

<400> 1

Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Glu Glu Phe Ile
65                  70                  75                  80

Met Leu Met Ala Arg Leu Thr Trp Ala Ser His Glu Lys Met His Glu
                85                  90                  95

Gly Asp Glu Gly Pro Gly His His His Lys Pro Gly Leu Gly Glu Gly
            100                 105                 110

Thr Pro

<210> 2
<211> 237
<212> PRT
<213> Homo sapiens

<400> 2

Met Asp Leu Trp Ser Ala Gly Cys Val Phe Tyr Glu Ile Ala Ser Leu
1               5                   10                  15

Gln Pro Leu Phe Pro Gly Val Asn Glu Leu Asp Gln Ile Ser Lys Ile
            20                  25                  30

His Asp Val Ile Gly Thr Pro Ala Gln Lys Ile Leu Thr Lys Phe Lys
        35                  40                  45

Gln Ser Arg Ala Met Asn Phe Asp Phe Pro Phe Lys Lys Gly Ser Gly
    50                  55                  60

Ile Pro Leu Leu Thr Thr Asn Leu Ser Pro Gln Cys Leu Ser Leu Leu
65                  70                  75                  80

His Ala Met Val Ala Tyr Asp Pro Asp Glu Arg Ile Ala Ala His Gln
                85                  90                  95

Ala Leu Gln His Pro Tyr Phe Gln Glu Gln Arg Lys Thr Glu Lys Arg
            100                 105                 110

Ala Leu Gly Ser His Arg Lys Ala Gly Phe Pro Glu His Pro Val Ala
        115                 120                 125

Pro Glu Pro Leu Ser Asn Ser Cys Gln Ile Ser Lys Glu Gly Arg Lys
    130                 135                 140

Gln Lys Gln Ser Leu Lys Gln Glu Glu Asp Arg Pro Lys Arg Arg Gly
145                 150                 155                 160

Pro Ala Tyr Val Met Glu Leu Pro Lys Leu Lys Leu Ser Gly Val Val
                165                 170                 175

Arg Leu Ser Ser Tyr Ser Ser Pro Thr Leu Gln Ser Val Leu Gly Ser
            180                 185                 190

Gly Thr Asn Gly Arg Val Pro Val Leu Arg Pro Leu Lys Cys Ile Pro
        195                 200                 205

Ala Ser Lys Lys Thr Asp Pro Gln Lys Asp Leu Lys Pro Ala Pro Gln
    210                 215                 220

Gln Cys Arg Leu Pro Thr Ile Val Arg Lys Gly Gly Arg
225                 230                 235

<210>  3
<211>  786
<212>  PRT
<213>  Homo sapiens

<400>  3

Met Thr Ser Ile Phe His Phe Ala Ile Ile Phe Met Leu Ile Leu Gln
1               5                   10                  15

Ile Arg Ile Gln Leu Ser Glu Glu Ser Glu Phe Leu Val Asp Arg Ser
            20                  25                  30

Lys Asn Gly Leu Ile His Val Pro Lys Asp Leu Ser Gln Lys Thr Thr
        35                  40                  45

Ile Leu Asn Ile Ser Gln Asn Tyr Ile Ser Glu Leu Trp Thr Ser Asp
    50                  55                  60

Ile Leu Ser Leu Ser Lys Leu Arg Ile Leu Ile Ile Ser His Asn Arg
65                  70                  75                  80

Ile Gln Tyr Leu Asp Ile Ser Val Phe Lys Phe Asn Gln Glu Leu Glu
                85                  90                  95

Tyr Leu Asp Leu Ser His Asn Lys Leu Val Lys Ile Ser Cys His Pro
            100                 105                 110

Thr Val Asn Leu Lys His Leu Asp Leu Ser Phe Asn Ala Phe Asp Ala
        115                 120                 125

Leu Pro Ile Cys Lys Glu Phe Gly Asn Met Ser Gln Leu Lys Phe Leu
    130                 135                 140

Gly Leu Ser Thr Thr His Leu Glu Lys Ser Ser Val Leu Pro Ile Ala
145                 150                 155                 160

His Leu Asn Ile Ser Lys Val Leu Leu Val Leu Gly Glu Thr Tyr Gly
                165                 170                 175

Glu Lys Glu Asp Pro Glu Gly Leu Gln Asp Phe Asn Thr Glu Ser Leu
            180                 185                 190

His Ile Val Phe Pro Thr Asn Lys Glu Phe His Phe Ile Leu Asp Val
        195                 200                 205

Ser Val Lys Thr Val Ala Asn Leu Glu Leu Ser Asn Ile Lys Cys Val
    210                 215                 220

Leu Glu Asp Asn Lys Cys Ser Tyr Phe Leu Ser Ile Leu Ala Lys Leu
225                 230                 235                 240

Gln Thr Asn Pro Lys Leu Ser Asn Leu Thr Leu Asn Asn Ile Glu Thr
                245                 250                 255

Thr Trp Asn Ser Phe Ile Arg Ile Leu Gln Leu Val Trp His Thr Thr
            260                 265                 270

Val Trp Tyr Phe Ser Ile Ser Asn Val Lys Leu Gln Gly Gln Leu Asp
        275                 280                 285

Phe Arg Asp Phe Asp Tyr Ser Gly Thr Ser Leu Lys Ala Leu Ser Ile
    290                 295                 300

His Gln Val Val Ser Asp Val Phe Gly Phe Pro Gln Ser Tyr Ile Tyr
305                 310                 315                 320

Glu Ile Phe Ser Asn Met Asn Ile Lys Asn Phe Thr Val Ser Gly Thr
                325                 330                 335

Arg Met Val His Met Leu Cys Pro Ser Lys Ile Ser Pro Phe Leu His
            340                 345                 350

Leu Asp Phe Ser Asn Asn Leu Leu Thr Asp Thr Val Phe Glu Asn Cys
        355                 360                 365

Gly His Leu Thr Glu Leu Glu Thr Leu Ile Leu Gln Met Asn Gln Leu
    370                 375                 380

Lys Glu Leu Ser Lys Ile Ala Glu Met Thr Thr Gln Met Lys Ser Leu
385                 390                 395                 400

Gln Gln Leu Asp Ile Ser Gln Asn Ser Val Ser Tyr Asp Glu Lys Lys
                405                 410                 415

Gly Asp Cys Ser Trp Thr Lys Ser Leu Leu Ser Leu Asn Met Ser Ser
            420                 425                 430

Asn Ile Leu Thr Asp Thr Ile Phe Arg Cys Leu Pro Pro Arg Ile Lys
        435                 440                 445

Val Leu Asp Leu His Ser Asn Lys Ile Lys Ser Ile Pro Lys Gln Val
    450                 455                 460

Val Lys Leu Glu Ala Leu Gln Glu Leu Asn Val Ala Phe Asn Ser Leu
465                 470                 475                 480

Thr Asp Leu Pro Gly Cys Gly Ser Phe Ser Ser Leu Ser Val Leu Ile
                485                 490                 495

Ile Asp His Asn Ser Val Ser His Pro Ser Ala Asp Phe Phe Gln Ser
            500                 505                 510

Cys Gln Lys Met Arg Ser Ile Lys Ala Gly Asp Asn Pro Phe Gln Cys
        515                 520                 525

Thr Cys Glu Leu Gly Glu Phe Val Lys Asn Ile Asp Gln Val Ser Ser
    530                 535                 540

Glu Val Leu Glu Gly Trp Pro Asp Ser Tyr Lys Cys Asp Tyr Pro Glu
545                 550                 555                 560

Ser Tyr Arg Gly Thr Leu Leu Lys Asp Phe His Met Ser Glu Leu Ser
                565                 570                 575

Cys Asn Ile Thr Leu Leu Ile Val Thr Ile Val Ala Thr Met Leu Val
            580                 585                 590

Leu Ala Val Thr Val Thr Ser Leu Cys Ser Tyr Leu Asp Leu Pro Trp
        595                 600                 605

Tyr Leu Arg Met Val Cys Gln Trp Thr Gln Thr Arg Arg Arg Ala Arg
    610                 615                 620

Asn Ile Pro Leu Glu Glu Leu Gln Arg Asn Leu Gln Phe His Ala Phe
625                 630                 635                 640

Ile Ser Tyr Ser Gly His Asp Ser Phe Trp Val Lys Asn Glu Leu Leu
                645                 650                 655

Pro Asn Leu Glu Lys Glu Gly Met Gln Ile Cys Leu His Glu Arg Asn
            660                 665                 670

Phe Val Pro Gly Lys Ser Ile Val Glu Asn Ile Ile Thr Cys Ile Glu
        675                 680                 685

Lys Ser Tyr Lys Ser Ile Phe Val Leu Ser Pro Asn Phe Val Gln Ser
    690                 695                 700

Glu Trp Cys His Tyr Glu Leu Tyr Phe Ala His His Asn Leu Phe His
705                 710                 715                 720

Glu Gly Ser Asn Ser Leu Ile Leu Ile Leu Leu Glu Pro Ile Pro Gln
                725                 730                 735

Tyr Ser Ile Pro Ser Ser Tyr His Lys Leu Lys Ser Leu Met Ala Arg
            740                 745                 750

Arg Thr Tyr Leu Glu Trp Pro Lys Glu Lys Ser Lys Arg Gly Leu Phe
        755                 760                 765

Trp Ala Asn Leu Arg Ala Ala Ile Asn Ile Lys Leu Thr Glu Gln Ala
    770                 775                 780

Lys Lys
785

<210>  4
<211>  586
<212>  PRT
<213>  Homo sapiens

<400>  4

Met Pro His Thr Leu Trp Met Val Trp Val Leu Gly Val Ile Ile Ser
1               5                   10                  15

Leu Ser Lys Glu Glu Ser Ser Asn Gln Ala Ser Leu Ser Cys Asp Arg
            20                  25                  30

Asn Gly Ile Cys Lys Gly Ser Ser Gly Ser Leu Asn Ser Ile Pro Ser
        35                  40                  45

Gly Leu Thr Glu Ala Val Lys Ser Leu Asp Gln Ser Asn Asn Arg Ile
    50                  55                  60

Thr Tyr Ile Cys Asn Ser Asp Leu Gln Arg Cys Val Asn Leu Gln Ala
65                  70                  75                  80

Leu Val Leu Thr Ser Asn Gly Ile Asn Thr Ile Glu Glu Asp Ser Phe
                85                  90                  95

Ser Ser Leu Gly Ser Leu Glu His Leu Asp Leu Ser Tyr Asn Tyr Leu

            100                 105                 110

Ser Asn Leu Ser Ser Ser Trp Phe Lys Pro Leu Ser Ser Leu Thr Phe
        115                 120                 125

Leu Asn Leu Leu Gly Asn Pro Tyr Lys Thr Leu Gly Glu Thr Ser Leu
    130                 135                 140

Phe Ser His Leu Thr Lys Leu Gln Ile Leu Arg Val Gly Asn Met Asp
145                 150                 155                 160

Thr Phe Thr Lys Ile Gln Arg Lys Asp Phe Ala Gly Leu Thr Phe Leu
                165                 170                 175

Glu Glu Leu Glu Ile Asp Ala Ser Asp Leu Gln Ser Tyr Glu Pro Lys
            180                 185                 190

Ser Leu Lys Ser Ile Gln Asn Val Ser His Leu Ile Leu His Met Lys
        195                 200                 205

Gln His Ile Leu Leu Leu Glu Ile Phe Val Asp Val Thr Ser Ser Val
    210                 215                 220

Glu Cys Leu Glu Leu Arg Asp Thr Asp Leu Asp Thr Phe His Phe Ser
225                 230                 235                 240

Glu Leu Ser Thr Gly Glu Thr Asn Ser Leu Ile Lys Lys Phe Thr Phe
                245                 250                 255

Arg Asn Val Lys Ile Thr Asp Glu Ser Leu Phe Gln Val Met Lys Leu
            260                 265                 270

Leu Asn Gln Ile Ser Gly Leu Leu Glu Leu Glu Phe Asp Asp Cys Thr
        275                 280                 285

Leu Asn Gly Val Gly Asn Phe Arg Ala Ser Asp Asn Asp Arg Val Ile
    290                 295                 300

Asp Pro Gly Lys Val Glu Thr Leu Thr Ile Arg Arg Leu His Ile Pro
305                 310                 315                 320

Arg Phe Tyr Leu Phe Tyr Asp Leu Ser Thr Leu Tyr Ser Leu Thr Glu
                325                 330                 335

Arg Val Lys Arg Ile Thr Val Glu Asn Ser Lys Val Phe Leu Val Pro

            340                 345                 350

Cys Leu Leu Ser Gln His Leu Lys Ser Leu Glu Tyr Leu Asp Leu Ser
        355                 360                 365

Glu Asn Leu Met Val Glu Glu Tyr Leu Lys Asn Ser Ala Cys Glu Asp
    370                 375                 380

Ala Trp Pro Ser Leu Gln Thr Leu Ile Leu Arg Gln Asn His Leu Ala
385                 390                 395                 400

Ser Leu Glu Lys Thr Gly Glu Thr Leu Leu Thr Leu Lys Asn Leu Thr
                405                 410                 415

Asn Ile Asp Ile Ser Lys Asn Ser Phe His Ser Met Pro Glu Thr Cys
            420                 425                 430

Gln Trp Pro Glu Lys Met Lys Tyr Leu Asn Leu Ser Ser Thr Arg Ile
        435                 440                 445

His Ser Val Thr Gly Cys Ile Pro Lys Thr Leu Glu Ile Leu Asp Val
    450                 455                 460

Ser Asn Asn Asn Leu Asn Leu Phe Ser Leu Asn Leu Pro Gln Leu Lys
465                 470                 475                 480

Glu Leu Tyr Ile Ser Arg Asn Lys Leu Met Thr Leu Pro Asp Ala Ser
                485                 490                 495

Leu Leu Pro Met Leu Leu Val Leu Lys Ile Ser Arg Asn Ala Ile Thr
            500                 505                 510

Thr Phe Ser Lys Glu Gln Leu Asp Ser Phe His Thr Leu Lys Thr Leu
        515                 520                 525

Glu Ala Gly Gly Asn Asn Phe Ile Cys Ser Cys Glu Phe Leu Ser Phe
    530                 535                 540

Thr Gln Glu Gln Gln Ala Leu Ala Lys Val Leu Ile Asp Trp Pro Ala
545                 550                 555                 560

Asn Tyr Leu Cys Asp Ser Pro Ser His Val Arg Gly Gln Gln Val Gln
                565                 570                 575

Asp Val Arg Leu Ser Val Ser Glu Cys His

580                 585

<210>  5
<211>  904
<212>  PRT
<213>  Homo sapiens

<400>  5

<210>  6
<211>  839

<212>  PRT
<213>  Homo sapiens

<400>  6

Met Met Ser Ala Ser Arg Leu Ala Gly Thr Leu Ile Pro Ala Met Ala
1               5                   10                  15

Phe Leu Ser Cys Val Arg Pro Glu Ser Trp Glu Pro Cys Val Glu Val
            20                  25                  30

Val Pro Asn Ile Thr Tyr Gln Cys Met Glu Leu Asn Phe Tyr Lys Ile
        35                  40                  45

Pro Asp Asn Leu Pro Phe Ser Thr Lys Asn Leu Asp Leu Ser Phe Asn
    50                  55                  60

Pro Leu Arg His Leu Gly Ser Tyr Ser Phe Phe Ser Phe Pro Glu Leu
65                  70                  75                  80

Gln Val Leu Asp Leu Ser Arg Cys Glu Ile Gln Thr Ile Glu Asp Gly
                85                  90                  95

Ala Tyr Gln Ser Leu Ser His Leu Ser Thr Leu Ile Leu Thr Gly Asn
            100                 105                 110

Pro Ile Gln Ser Leu Ala Leu Gly Ala Phe Ser Gly Leu Ser Ser Leu
        115                 120                 125

Gln Lys Leu Val Ala Val Glu Thr Asn Leu Ala Ser Leu Glu Asn Phe
    130                 135                 140

Pro Ile Gly His Leu Lys Thr Leu Lys Glu Leu Asn Val Ala His Asn
145                 150                 155                 160

Leu Ile Gln Ser Phe Lys Leu Pro Glu Tyr Phe Ser Asn Leu Thr Asn
                165                 170                 175

Leu Glu His Leu Asp Leu Ser Ser Asn Lys Ile Gln Ser Ile Tyr Cys
            180                 185                 190

Thr Asp Leu Arg Val Leu His Gln Met Pro Leu Leu Asn Leu Ser Leu
        195                 200                 205

Asp Leu Ser Leu Asn Pro Met Asn Phe Ile Gln Pro Gly Ala Phe Lys
    210                 215                 220

Glu Ile Arg Leu His Lys Leu Thr Leu Arg Asn Asn Phe Asp Ser Leu
225                 230                 235                 240

Asn Val Met Lys Thr Cys Ile Gln Gly Leu Ala Gly Leu Glu Val His
                245                 250                 255

Arg Leu Val Leu Gly Glu Phe Arg Asn Glu Gly Asn Leu Glu Lys Phe
            260                 265                 270

Asp Lys Ser Ala Leu Glu Gly Leu Cys Asn Leu Thr Ile Glu Glu Phe
        275                 280                 285

Arg Leu Ala Tyr Leu Asp Tyr Tyr Leu Asp Asp Ile Ile Asp Leu Phe
    290                 295                 300

Asn Cys Leu Thr Asn Val Ser Ser Phe Ser Leu Val Ser Val Thr Ile
305                 310                 315                 320

Glu Arg Val Lys Asp Phe Ser Tyr Asn Phe Gly Trp Gln His Leu Glu
                325                 330                 335

Leu Val Asn Cys Lys Phe Gly Gln Phe Pro Thr Leu Lys Leu Lys Ser
            340                 345                 350

Leu Lys Arg Leu Thr Phe Thr Ser Asn Lys Gly Gly Asn Ala Phe Ser
        355                 360                 365

Glu Val Asp Leu Pro Ser Leu Glu Phe Leu Asp Leu Ser Arg Asn Gly
    370                 375                 380

Leu Ser Phe Lys Gly Cys Cys Ser Gln Ser Asp Phe Gly Thr Thr Ser
385                 390                 395                 400

Leu Lys Tyr Leu Asp Leu Ser Phe Asn Gly Val Ile Thr Met Ser Ser
                405                 410                 415

Asn Phe Leu Gly Leu Glu Gln Leu Glu His Leu Asp Phe Gln His Ser
            420                 425                 430

Asn Leu Lys Gln Met Ser Glu Phe Ser Val Phe Leu Ser Leu Arg Asn
        435                 440                 445

Leu Ile Tyr Leu Asp Ile Ser His Thr His Thr Arg Val Ala Phe Asn
    450                 455                 460

Gly Ile Phe Asn Gly Leu Ser Ser Leu Glu Val Leu Lys Met Ala Gly
465                 470                 475                 480

Asn Ser Phe Gln Glu Asn Phe Leu Pro Asp Ile Phe Thr Glu Leu Arg
                485                 490                 495

Asn Leu Thr Phe Leu Asp Leu Ser Gln Cys Gln Leu Glu Gln Leu Ser
            500                 505                 510

Pro Thr Ala Phe Asn Ser Leu Ser Ser Leu Gln Val Leu Asn Met Ser
        515                 520                 525

His Asn Asn Phe Phe Ser Leu Asp Thr Phe Pro Tyr Lys Cys Leu Asn
    530                 535                 540

Ser Leu Gln Val Leu Asp Tyr Ser Leu Asn His Ile Met Thr Ser Lys
545                 550                 555                 560

Lys Gln Glu Leu Gln His Phe Pro Ser Ser Leu Ala Phe Leu Asn Leu
                565                 570                 575

Thr Gln Asn Asp Phe Ala Cys Thr Cys Glu His Gln Ser Phe Leu Gln
            580                 585                 590

Trp Ile Lys Asp Gln Arg Gln Leu Leu Val Glu Val Glu Arg Met Glu
        595                 600                 605

Cys Ala Thr Pro Ser Asp Lys Gln Gly Met Pro Val Leu Ser Leu Asn
    610                 615                 620

Ile Thr Cys Gln Met Asn Lys Thr Ile Ile Gly Val Ser Val Leu Ser
625                 630                 635                 640

Val Leu Val Val Ser Val Val Ala Val Leu Val Tyr Lys Phe Tyr Phe
                645                 650                 655

His Leu Met Leu Leu Ala Gly Cys Ile Lys Tyr Gly Arg Gly Glu Asn
            660                 665                 670

Ile Tyr Asp Ala Phe Val Ile Tyr Ser Ser Gln Asp Glu Asp Trp Val
        675                 680                 685

Arg Asn Glu Leu Val Lys Asn Leu Glu Glu Gly Val Pro Pro Phe Gln
    690                 695                 700

Leu Cys Leu His Tyr Arg Asp Phe Ile Pro Gly Val Ala Ile Ala Ala
705                 710                 715                 720

Asn Ile Ile His Glu Gly Phe His Lys Ser Arg Lys Val Ile Val Val
                725                 730                 735

Val Ser Gln His Phe Ile Gln Ser Arg Trp Cys Ile Phe Glu Tyr Glu
            740                 745                 750

Ile Ala Gln Thr Trp Gln Phe Leu Ser Ser Arg Ala Gly Ile Ile Phe
        755                 760                 765

Ile Val Leu Gln Lys Val Glu Lys Thr Leu Leu Arg Gln Gln Val Glu
    770                 775                 780

Leu Tyr Arg Leu Leu Ser Arg Asn Thr Tyr Leu Glu Trp Glu Asp Ser
785                 790                 795                 800

Val Leu Gly Arg His Ile Phe Trp Arg Arg Leu Arg Lys Ala Leu Leu
                805                 810                 815

Asp Gly Lys Ser Trp Asn Pro Glu Gly Thr Val Gly Thr Gly Cys Asn
            820                 825                 830

Trp Gln Glu Ala Thr Ser Ile
        835

<210>  7
<211>  858
<212>  PRT
<213>  Homo sapiens

<400>  7

<210>  8
<211>  796
<212>  PRT
<213>  Homo sapiens

<400>  8

Met Thr Lys Asp Lys Glu Pro Ile Val Lys Ser Phe His Phe Val Cys
1               5                   10                  15

Leu Met Ile Ile Ile Val Gly Thr Arg Ile Gln Phe Ser Asp Gly Asn
            20                  25                  30

Glu Phe Ala Val Asp Lys Ser Lys Arg Gly Leu Ile His Val Pro Lys
        35                  40                  45

Asp Leu Pro Leu Lys Thr Lys Val Leu Asp Met Ser Gln Asn Tyr Ile
    50                  55                  60

Ala Glu Leu Gln Val Ser Asp Met Ser Phe Leu Ser Glu Leu Thr Val
65                  70                  75                  80

Leu Arg Leu Ser His Asn Arg Ile Gln Leu Leu Asp Leu Ser Val Phe
                85                  90                  95

Lys Phe Asn Gln Asp Leu Glu Tyr Leu Asp Leu Ser His Asn Gln Leu
            100                 105                 110

Gln Lys Ile Ser Cys His Pro Ile Val Ser Phe Arg His Leu Asp Leu
        115                 120                 125

Ser Phe Asn Asp Phe Lys Ala Leu Pro Ile Cys Lys Glu Phe Gly Asn

130                 135                 140

Leu Ser Gln Leu Asn Phe Leu Gly Leu Ser Ala Met Lys Leu Gln Lys
145                 150                 155                 160

Leu Asp Leu Leu Pro Ile Ala His Leu His Leu Ser Tyr Ile Leu Leu
                165                 170                 175

Asp Leu Arg Asn Tyr Tyr Ile Lys Glu Asn Glu Thr Glu Ser Leu Gln
            180                 185                 190

Ile Leu Asn Ala Lys Thr Leu His Leu Val Phe His Pro Thr Ser Leu
        195                 200                 205

Phe Ala Ile Gln Val Asn Ile Ser Val Asn Thr Leu Gly Cys Leu Gln
    210                 215                 220

Leu Thr Asn Ile Lys Leu Asn Asp Asp Asn Cys Gln Val Phe Ile Lys
225                 230                 235                 240

Phe Leu Ser Glu Leu Thr Arg Gly Ser Thr Leu Leu Asn Phe Thr Leu
                245                 250                 255

Asn His Ile Glu Thr Thr Trp Lys Cys Leu Val Arg Val Phe Gln Phe
            260                 265                 270

Leu Trp Pro Lys Pro Val Glu Tyr Leu Asn Ile Tyr Asn Leu Thr Ile
        275                 280                 285

Ile Glu Ser Ile Arg Glu Glu Asp Phe Thr Tyr Ser Lys Thr Thr Leu
    290                 295                 300

Lys Ala Leu Thr Ile Glu His Ile Thr Asn Gln Val Phe Leu Phe Ser
305                 310                 315                 320

Gln Thr Ala Leu Tyr Thr Val Phe Ser Glu Met Asn Ile Met Met Leu
                325                 330                 335

Thr Ile Ser Asp Thr Pro Phe Ile His Met Leu Cys Pro His Ala Pro
            340                 345                 350

Ser Thr Phe Lys Phe Leu Asn Phe Thr Gln Asn Val Phe Thr Asp Ser
        355                 360                 365

Ile Phe Glu Lys Cys Ser Thr Leu Val Lys Leu Glu Thr Leu Ile Leu

370                 375                 380

Gln Lys Asn Gly Leu Lys Asp Leu Phe Lys Val Gly Leu Met Thr Lys
385                 390                 395                 400

Asp Met Pro Ser Leu Glu Ile Leu Asp Val Ser Trp Asn Ser Leu Glu
                405                 410                 415

Ser Gly Arg His Lys Glu Asn Cys Thr Trp Val Glu Ser Ile Val Val
            420                 425                 430

Leu Asn Leu Ser Ser Asn Met Leu Thr Asp Ser Val Phe Arg Cys Leu
        435                 440                 445

Pro Pro Arg Ile Lys Val Leu Asp Leu His Ser Asn Lys Ile Lys Ser
    450                 455                 460

Val Pro Lys Gln Val Val Lys Leu Glu Ala Leu Gln Glu Leu Asn Val
465                 470                 475                 480

Ala Phe Asn Ser Leu Thr Asp Leu Pro Gly Cys Gly Ser Phe Ser Ser
                485                 490                 495

Leu Ser Val Leu Ile Ile Asp His Asn Ser Val Ser His Pro Ser Ala
            500                 505                 510

Asp Phe Phe Gln Ser Cys Gln Lys Met Arg Ser Ile Lys Ala Gly Asp
        515                 520                 525

Asn Pro Phe Gln Cys Thr Cys Glu Leu Arg Glu Phe Val Lys Asn Ile
    530                 535                 540

Asp Gln Val Ser Ser Glu Val Leu Glu Gly Trp Pro Asp Ser Tyr Lys
545                 550                 555                 560

Cys Asp Tyr Pro Glu Ser Tyr Arg Gly Ser Pro Leu Lys Asp Phe His
                565                 570                 575

Met Ser Glu Leu Ser Cys Asn Ile Thr Leu Leu Ile Val Thr Ile Gly
            580                 585                 590

Ala Thr Met Leu Val Leu Ala Val Thr Val Thr Ser Leu Cys Ile Tyr
        595                 600                 605

Leu Asp Leu Pro Trp Tyr Leu Arg Met Val Cys Gln Trp Thr Gln Thr

610                 615                 620

Arg Arg Arg Ala Arg Asn Ile Pro Leu Glu Glu Leu Gln Arg Asn Leu
625                 630                 635                 640

Gln Phe His Ala Phe Ile Ser Tyr Ser Glu His Asp Ser Ala Trp Val
                645                 650                 655

Lys Ser Glu Leu Val Pro Tyr Leu Glu Lys Glu Asp Ile Gln Ile Cys
            660                 665                 670

Leu His Glu Arg Asn Phe Val Pro Gly Lys Ser Ile Val Glu Asn Ile
        675                 680                 685

Ile Asn Cys Ile Glu Lys Ser Tyr Lys Ser Ile Phe Val Leu Ser Pro
    690                 695                 700

Asn Phe Val Gln Ser Glu Trp Cys His Tyr Glu Leu Tyr Phe Ala His
705                 710                 715                 720

His Asn Leu Phe His Glu Gly Ser Asn Asn Leu Ile Leu Ile Leu Leu
                725                 730                 735

Glu Pro Ile Pro Gln Asn Ser Ile Pro Asn Lys Tyr His Lys Leu Lys
            740                 745                 750

Ala Leu Met Thr Gln Arg Thr Tyr Leu Gln Trp Pro Lys Glu Lys Ser
        755                 760                 765

Lys Arg Gly Leu Phe Trp Ala Asn Ile Arg Ala Ala Phe Asn Met Lys
    770                 775                 780

Leu Thr Leu Val Thr Glu Asn Asn Asp Val Lys Ser
785                 790                 795

<210>  9
<211>  1049
<212>  PRT
<213>  Homo sapiens

<400>  9

<210> 10
<211> 1059
<212> PRT
<213> Homo sapiens

<400> 10

<210>  11
<211>  1032
<212>  PRT
<213>  Homo sapiens

<400>  11

<210>  12
<211>  811
<212>  PRT
<213>  Homo sapiens

<400>  12

Met Arg Leu Ile Arg Asn Ile Tyr Ile Phe Cys Ser Ile Val Met Thr
1               5                   10                  15

Ala Glu Gly Asp Ala Pro Glu Leu Pro Glu Glu Arg Glu Leu Met Thr
            20                  25                  30

Asn Cys Ser Asn Met Ser Leu Arg Lys Val Pro Ala Asp Leu Thr Pro
        35                  40                  45

Ala Thr Thr Thr Leu Asp Leu Ser Tyr Asn Leu Leu Phe Gln Leu Gln
    50                  55                  60

Ser Ser Asp Phe His Ser Val Ser Lys Leu Arg Val Leu Ile Leu Cys
65                  70                  75                  80

His Asn Arg Ile Gln Gln Leu Asp Leu Lys Thr Phe Glu Phe Asn Lys
                85                  90                  95

Glu Leu Arg Tyr Leu Asp Leu Ser Asn Asn Arg Leu Lys Ser Val Thr
            100                 105                 110

Trp Tyr Leu Leu Ala Gly Leu Arg Tyr Leu Asp Leu Ser Phe Asn Asp
        115                 120                 125

Phe Asp Thr Met Pro Ile Cys Glu Glu Ala Gly Asn Met Ser His Leu

130                 135                 140

Glu Ile Leu Gly Leu Ser Gly Ala Lys Ile Gln Lys Ser Asp Phe Gln
145                 150                 155                 160

Lys Ile Ala His Leu His Leu Asn Thr Val Phe Leu Gly Phe Arg Thr
                165                 170                 175

Leu Pro His Tyr Glu Glu Gly Ser Leu Pro Ile Leu Asn Thr Thr Lys
            180                 185                 190

Leu His Ile Val Leu Pro Met Asp Thr Asn Phe Trp Val Leu Leu Arg
        195                 200                 205

Asp Gly Ile Lys Thr Ser Lys Ile Leu Glu Met Thr Asn Ile Asp Gly
    210                 215                 220

Lys Ser Gln Phe Val Ser Tyr Glu Met Gln Arg Asn Leu Ser Leu Glu
225                 230                 235                 240

Asn Ala Lys Thr Ser Val Leu Leu Leu Asn Lys Val Asp Leu Leu Trp
                245                 250                 255

Asp Asp Leu Phe Leu Ile Leu Gln Phe Val Trp His Thr Ser Val Glu
            260                 265                 270

His Phe Gln Ile Arg Asn Val Thr Phe Gly Gly Lys Ala Tyr Leu Asp
        275                 280                 285

His Asn Ser Phe Asp Tyr Ser Asn Thr Val Met Arg Thr Ile Lys Leu
    290                 295                 300

Glu His Val His Phe Arg Val Phe Tyr Ile Gln Gln Asp Lys Ile Tyr
305                 310                 315                 320

Leu Leu Leu Thr Lys Met Asp Ile Glu Asn Leu Thr Ile Ser Asn Ala
                325                 330                 335

Gln Met Pro His Met Leu Phe Pro Asn Tyr Pro Thr Lys Phe Gln Tyr
            340                 345                 350

Leu Asn Phe Ala Asn Asn Ile Leu Thr Asp Glu Leu Phe Lys Arg Thr
        355                 360                 365

Ile Gln Leu Pro His Leu Lys Thr Leu Ile Leu Asn Gly Asn Lys Leu

370                 375                 380

Glu Thr Leu Ser Leu Val Ser Cys Phe Ala Asn Asn Thr Pro Leu Glu
385                 390                 395                 400

His Leu Asp Leu Ser Gln Asn Leu Leu Gln His Lys Asn Asp Glu Asn
                405                 410                 415

Cys Ser Trp Pro Glu Thr Val Val Asn Met Asn Leu Ser Tyr Asn Lys
            420                 425                 430

Leu Ser Asp Ser Val Phe Arg Cys Leu Pro Lys Ser Ile Gln Ile Leu
        435                 440                 445

Asp Leu Asn Asn Asn Gln Ile Gln Thr Val Pro Lys Glu Thr Ile His
    450                 455                 460

Leu Met Ala Leu Arg Glu Leu Asn Ile Ala Phe Asn Phe Leu Thr Asp
465                 470                 475                 480

Leu Pro Gly Cys Ser His Phe Ser Arg Leu Ser Val Leu Asn Ile Glu
                485                 490                 495

Met Asn Phe Ile Leu Ser Pro Ser Leu Asp Phe Val Gln Ser Cys Gln
            500                 505                 510

Glu Val Lys Thr Leu Asn Ala Gly Arg Asn Pro Phe Arg Cys Thr Cys
        515                 520                 525

Glu Leu Lys Asn Phe Ile Gln Leu Glu Thr Tyr Ser Glu Val Met Met
    530                 535                 540

Val Gly Trp Ser Asp Ser Tyr Thr Cys Glu Tyr Pro Leu Asn Leu Arg
545                 550                 555                 560

Gly Ile Arg Leu Lys Asp Val His Leu His Glu Leu Ser Cys Asn Thr
                565                 570                 575

Ala Leu Leu Ile Val Thr Ile Val Val Ile Met Leu Val Leu Gly Leu
            580                 585                 590

Ala Val Ala Phe Cys Cys Leu His Phe Asp Leu Pro Trp Tyr Leu Arg
        595                 600                 605

Met Leu Gly Gln Cys Thr Gln Thr Trp His Arg Val Arg Lys Thr Thr

610                 615                 620

Gln Glu Gln Leu Lys Arg Asn Val Arg Phe His Ala Phe Ile Ser Tyr
625                 630                 635                 640

Ser Glu His Asp Ser Leu Trp Val Lys Asn Glu Leu Ile Pro Asn Leu
                645                 650                 655

Glu Lys Glu Asp Gly Ser Ile Leu Ile Cys Leu Tyr Glu Ser Tyr Phe
            660                 665                 670

Asp Pro Gly Lys Ser Ile Ser Glu Asn Ile Val Ser Phe Ile Glu Lys
        675                 680                 685

Ser Tyr Lys Ser Ile Phe Val Leu Ser Pro Asn Phe Val Gln Asn Glu
    690                 695                 700

Trp Cys His Tyr Glu Phe Tyr Phe Ala His His Asn Leu Phe His Glu
705                 710                 715                 720

Asn Ser Asp His Ile Ile Leu Ile Leu Leu Glu Pro Ile Pro Phe Tyr
                725                 730                 735

Cys Ile Pro Thr Arg Tyr His Lys Leu Lys Ala Leu Leu Glu Lys Lys
            740                 745                 750

Ala Tyr Leu Glu Trp Pro Lys Asp Arg Arg Lys Cys Gly Leu Phe Trp
        755                 760                 765

Ala Asn Leu Arg Ala Ala Ile Asn Val Asn Val Leu Ala Thr Arg Glu
    770                 775                 780

Met Tyr Glu Leu Gln Thr Phe Thr Glu Leu Asn Glu Glu Ser Arg Gly
785                 790                 795                 800

Ser Thr Ile Ser Leu Met Arg Thr Asp Cys Leu
                805                 810

<210>  13
<211>  926
<212>  PRT
<213>  Mus musculus

<400>  13

Met Pro Arg Met Glu Arg His Gln Phe Cys Ser Val Leu Leu Ile Leu
1               5                   10                  15

Ile Leu Leu Thr Leu Val Ser Leu Thr Leu Thr Gly Trp Ala Trp Thr
            20                  25                  30

Ile Pro Asp Cys Ile Ile Ala Asp Ser Leu Leu Phe Pro Asn Leu Ser
        35                  40                  45

Tyr Tyr Ile Pro Phe Cys Thr Ser Ala Pro Gly Leu His Leu Leu Ala
    50                  55                  60

Ser Cys Ser Asn Val Lys Asn Leu Asn Gln Thr Leu Lys Arg Val Pro
65                  70                  75                  80

Arg Asn Thr Glu Val Leu Cys Leu Gln Gly Met Val Pro Thr Leu Pro
                85                  90                  95

Ala Lys Ala Phe Ile Arg Phe His Ser Leu Gln Leu Leu Arg Leu Gln
            100                 105                 110

Leu Ser Thr Thr Ser Val Thr Ser Arg Thr Phe Gln Gly Leu Asp Gln
        115                 120                 125

Leu Gln Tyr Leu Phe Phe Asp His His Ala Pro Cys Cys Leu Ser Leu
    130                 135                 140

Phe Leu Ser Pro Asn Cys Phe Glu Ser Leu Arg Ser Leu Ser Ser Leu
145                 150                 155                 160

Ser Phe Gln Gly Tyr Cys Leu Thr Tyr Ser Gln Ser Ile Tyr Leu Pro
                165                 170                 175

Thr Ser Leu Arg His Leu Thr Leu Arg Asn Ser Cys Leu Thr Lys Phe
            180                 185                 190

Gln Asp Leu Gln Arg Leu Phe Pro Asp Leu Leu Leu Ser Thr Ser Ser
        195                 200                 205

Thr Pro Asn Thr Lys Pro Gly Ala Pro Phe Leu Glu Met Leu Asp Leu
    210                 215                 220

Ser Tyr Asn Leu Gln Leu Lys Gln Ala Gly Val Arg Asp Leu Tyr Gly
225                 230                 235                 240

Leu Thr Leu His Ser Leu Ile Leu Asp Gly Thr Pro Leu Arg Ala Leu
                245                 250                 255

Asp Leu Thr Asp Ser Gly Leu Leu His Leu His Phe Leu Ser Leu Val
            260                 265                 270

Gly Thr Gly Ile Glu Lys Val Pro Ala Ser Leu Thr Gly Tyr Ser Glu
        275                 280                 285

Leu Arg Ala Leu Asp Leu Gly Lys Asn Gln Ile Gln Asn Ile Leu Glu
    290                 295                 300

Asn Gly Glu Ile Pro Gly Tyr Lys Ala Leu Glu Phe Leu Ser Leu His
305                 310                 315                 320

Asp Asn His Leu Gln Thr Leu Pro Thr Arg Phe Leu His Thr Leu Pro
                325                 330                 335

Gln Leu Gln Lys Leu Asn Leu Ser Met Asn Lys Leu Gly Pro Ile Leu
            340                 345                 350

Glu Leu Pro Glu Gly Leu Phe Ser Thr Asn Leu Lys Val Leu Asp Leu
        355                 360                 365

Ser His Asn Gln Leu Cys Asp Val Pro His Gly Ala Phe Ser Leu Leu
    370                 375                 380

Ser Gln Leu Gln Glu Leu Trp Leu Ser Gly Asn Asn Ile Ser Ser Leu
385                 390                 395                 400

Ser Asn Glu Ser Leu Gln Gly Leu Arg Gln Leu Arg Thr Leu Asp Leu
                405                 410                 415

Ser Trp Asn Gln Ile Lys Val Leu Lys Pro Gly Trp Leu Ser His Leu
            420                 425                 430

Pro Ala Leu Thr Thr Leu Asn Leu Leu Gly Thr Tyr Leu Glu Asn Ile
        435                 440                 445

Leu Gly Ile Gln Leu Gln Gly Pro Lys Met Leu Arg His Leu Gln Leu
    450                 455                 460

Gly Ser Tyr Pro Met Leu Asp Ile Tyr Pro Pro Trp Pro Pro Thr Leu
465                 470                 475                 480

Leu Ser Leu Glu Ile Gln Ala Glu Ser Cys Ile Gln Phe Met Ile His
                485                 490                 495

Ser Gly Gln Pro Phe Leu Phe Leu Glu Asn Leu Thr Leu Glu Thr Ser
            500                 505                 510

Ile Leu Leu Leu Lys Pro Asp Asn Ile Thr Ile His Phe Pro Ser Leu
        515                 520                 525

Arg Arg Leu Thr Leu Arg Gly Tyr Ser Phe Ile Phe Ser Thr Ser Gln
    530                 535                 540

Leu Gln Arg Phe Phe Pro Gln Gln Leu Pro Leu Leu Glu His Phe Phe
545                 550                 555                 560

Ile Trp Cys Glu Asn Ser Tyr Ala Val Asp Leu Tyr Leu Phe Gly Met
                565                 570                 575

Pro Arg Leu Arg Val Leu Glu Leu Gly Tyr Leu Asn Phe Phe Tyr Glu
            580                 585                 590

Ser Ser Thr Met Lys Leu Glu Met Leu Leu Lys Glu Val Pro Gln Leu
        595                 600                 605

Gln Val Leu Ala Leu Ser His Leu Asn Leu Arg Asn Leu Ser Val Ser
    610                 615                 620

Ser Phe Lys Ser Leu Gln Asp Leu Lys Leu Leu Leu Phe Asn Ser Glu
625                 630                 635                 640

Arg Ala Leu Glu Met Asn Ser Asn Leu Gln Glu Phe Ile Pro Gln Met
                645                 650                 655

Pro Gln Tyr Val Tyr Phe Ser Asp Val Thr Phe Thr Cys Gln Cys Glu
            660                 665                 670

Ala Ser Trp Leu Glu Ser Trp Ala Thr Arg Ala Pro Asn Thr Phe Val
        675                 680                 685

Tyr Gly Leu Glu Lys Ser Ile Cys Ile Ala Asn Ala Ser Asp Tyr Ser
    690                 695                 700

Lys Thr Leu Leu Phe Ser Phe Leu Ala Thr Asn Cys Pro His Gly Thr
705                 710                 715                 720

Glu Phe Trp Gly Phe Leu Thr Ser Phe Ile Leu Leu Leu Leu Leu Ile
                725                 730                 735

Ile Leu Pro Leu Ile Ser Cys Pro Lys Trp Ser Trp Leu His His Leu
            740                 745                 750

Trp Thr Leu Phe His Thr Cys Trp Trp Lys Leu Cys Gly His Arg Leu
        755                 760                 765

Arg Gly Gln Phe Asn Tyr Asp Val Phe Ile Ser Tyr Cys Glu Glu Asp
    770                 775                 780

Gln Ala Trp Val Leu Glu Glu Leu Val Pro Val Leu Glu Lys Ala Pro
785                 790                 795                 800

Pro Glu Gly Glu Gly Leu Arg Leu Cys Leu Pro Ala Arg Asp Phe Gly
                805                 810                 815

Ile Gly Asn Asp Arg Met Glu Ser Met Ile Ala Ser Met Gly Lys Ser
            820                 825                 830

Arg Ala Thr Leu Cys Val Leu Thr Gly Gln Ala Leu Ala Ser Pro Trp
        835                 840                 845

Cys Asn Leu Glu Leu Arg Leu Ala Thr Tyr His Leu Val Ala Arg Pro
    850                 855                 860

Gly Thr Thr His Leu Leu Leu Leu Phe Leu Glu Pro Leu Asp Arg Gln
865                 870                 875                 880

Arg Leu His Ser Tyr His Arg Leu Ser Arg Trp Leu Gln Lys Glu Asp
                885                 890                 895

Tyr Phe Asp Leu Ser Gln Gly Lys Val Glu Trp Asn Ser Phe Cys Glu
            900                 905                 910

Gln Leu Lys Arg Arg Leu Ser Lys Ala Gly Gln Glu Arg Asp
        915                 920                 925

<210>  14
<211>  906
<212>  PRT
<213>  Mus musculus

<400>  14

Met Gly Arg Tyr Trp Leu Leu Pro Gly Leu Leu Leu Ser Leu Pro Leu
1               5                   10                  15

Val Thr Gly Trp Ser Thr Ser Asn Cys Leu Val Thr Glu Gly Ser Arg
            20                  25                  30

Leu Pro Leu Val Ser Arg Tyr Phe Thr Phe Cys Arg His Ser Lys Leu
        35                  40                  45

Ser Phe Leu Ala Ala Cys Leu Ser Val Ser Asn Leu Thr Gln Thr Leu
    50                  55                  60

Glu Val Val Pro Arg Thr Val Glu Gly Leu Cys Leu Gly Gly Thr Val
65                  70                  75                  80

Ser Thr Leu Leu Pro Asp Ala Phe Ser Ala Phe Pro Gly Leu Lys Val
                85                  90                  95

Leu Ala Leu Ser Leu His Leu Thr Gln Leu Leu Pro Gly Ala Leu Arg
            100                 105                 110

Gly Leu Gly Gln Leu Gln Ser Leu Ser Phe Phe Asp Ser Pro Leu Arg
        115                 120                 125

Arg Ser Leu Phe Leu Pro Pro Asp Ala Phe Ser Asp Leu Ile Ser Leu
    130                 135                 140

Gln Arg Leu His Ile Ser Gly Pro Cys Leu Asp Lys Lys Ala Gly Ile
145                 150                 155                 160

Arg Leu Pro Pro Gly Leu Gln Trp Leu Gly Val Thr Leu Ser Cys Ile
                165                 170                 175

Gln Asp Val Gly Glu Leu Ala Gly Met Phe Pro Asp Leu Val Gln Gly
            180                 185                 190

Ser Ser Ser Arg Val Ser Trp Thr Leu Gln Lys Leu Asp Leu Ser Ser
        195                 200                 205

Asn Trp Lys Leu Lys Met Ala Ser Pro Gly Ser Leu Gln Gly Leu Gln
    210                 215                 220

Val Glu Ile Leu Asp Leu Thr Arg Thr Pro Leu Asp Ala Val Trp Leu
225                 230                 235                 240

Lys Gly Leu Gly Leu Gln Lys Leu Asp Val Leu Tyr Ala Gln Thr Ala
                245                 250                 255

Thr Ala Glu Leu Ala Ala Glu Ala Val Ala His Phe Glu Leu Gln Gly
            260                 265                 270

Leu Ile Val Lys Glu Ser Lys Ile Gly Ser Ile Ser Gln Glu Ala Leu
        275                 280                 285

Ala Ser Cys His Ser Leu Lys Thr Leu Gly Leu Ser Ser Thr Gly Leu
    290                 295                 300

Thr Lys Leu Pro Pro Gly Phe Leu Thr Ala Met Pro Arg Leu Gln Arg
305                 310                 315                 320

Leu Glu Leu Ser Gly Asn Gln Leu Gln Ser Ala Val Leu Cys Met Asn
                325                 330                 335

Glu Thr Gly Asp Val Ser Gly Leu Thr Thr Leu Asp Leu Ser Gly Asn
            340                 345                 350

Arg Leu Arg Ile Leu Pro Pro Ala Ala Phe Ser Cys Leu Pro His Leu
        355                 360                 365

Arg Glu Leu Leu Leu Arg Tyr Asn Gln Leu Leu Ser Leu Glu Gly Tyr
    370                 375                 380

Leu Phe Gln Glu Leu Gln Gln Leu Glu Thr Leu Lys Leu Asp Gly Asn
385                 390                 395                 400

Pro Leu Leu His Leu Gly Lys Asn Trp Leu Ala Ala Leu Pro Ala Leu
                405                 410                 415

Thr Thr Leu Ser Leu Leu Asp Thr Gln Ile Arg Met Ser Pro Glu Pro
            420                 425                 430

Gly Phe Trp Gly Ala Lys Asn Leu His Thr Leu Ser Leu Lys Leu Pro
        435                 440                 445

Ala Leu Pro Ala Pro Ala Val Leu Phe Leu Pro Met Tyr Leu Thr Ser
    450                 455                 460

Leu Glu Leu His Ile Ala Ser Gly Thr Thr Glu His Trp Thr Leu Ser
465                 470                 475                 480

Pro Ala Ile Phe Pro Ser Leu Glu Thr Leu Thr Ile Ser Gly Gly Gly
                485                 490                 495

Leu Lys Leu Lys Leu Gly Ser Gln Asn Ala Ser Gly Val Phe Pro Ala
            500                 505                 510

Leu Gln Lys Leu Ser Leu Leu Lys Asn Ser Leu Asp Ala Phe Cys Ser
        515                 520                 525

Gln Gly Thr Ser Asn Leu Phe Leu Trp Gln Leu Pro Lys Leu Gln Ser
    530                 535                 540

Leu Arg Val Trp Gly Ala Gly Asn Ser Ser Arg Pro Cys Leu Ile Thr
545                 550                 555                 560

Gly Leu Pro Ser Leu Arg Glu Leu Lys Leu Ala Ser Leu Gln Ser Ile
                565                 570                 575

Thr Gln Pro Arg Ser Val Gln Leu Glu Glu Leu Val Gly Asp Leu Pro
            580                 585                 590

Gln Leu Gln Ala Leu Val Leu Ser Ser Thr Gly Leu Lys Ser Leu Ser
        595                 600                 605

Ala Ala Ala Phe Gln Arg Leu His Ser Leu Gln Val Leu Val Leu Glu
    610                 615                 620

Tyr Glu Lys Asp Leu Met Leu Gln Asp Ser Leu Arg Glu Tyr Ser Pro
625                 630                 635                 640

Gln Met Pro His Tyr Ile Tyr Ile Leu Glu Ser Asn Leu Ala Cys His
                645                 650                 655

Cys Ala Asn Ala Trp Met Glu Pro Trp Val Lys Arg Ser Thr Lys Thr
            660                 665                 670

Tyr Ile Tyr Ile Arg Asp Asn Arg Leu Cys Pro Gly Gln Asp Arg Leu
        675                 680                 685

Ser Ala Arg Gly Ser Leu Pro Ser Phe Leu Trp Asp His Cys Pro Gln
    690                 695                 700

Thr Leu Glu Leu Lys Leu Phe Leu Ala Ser Ser Ala Leu Val Phe Met
705                 710                 715                 720

Leu Ile Ala Leu Pro Leu Leu Gln Glu Ala Arg Asn Ser Trp Ile Pro
                725                 730                 735

Tyr Leu Gln Ala Leu Phe Arg Val Trp Leu Gln Gly Leu Arg Gly Lys
            740                 745                 750

Gly Asp Lys Gly Lys Arg Phe Leu Phe Asp Val Phe Val Ser His Cys
        755                 760                 765

Arg Gln Asp Gln Gly Trp Val Ile Glu Glu Leu Leu Pro Ala Leu Glu
    770                 775                 780

Gly Phe Leu Pro Ala Gly Leu Gly Leu Arg Leu Cys Leu Pro Glu Arg
785                 790                 795                 800

Asp Phe Glu Pro Gly Lys Asp Val Val Asp Asn Val Val Asp Ser Met
                805                 810                 815

Leu Ser Ser Arg Thr Thr Leu Cys Val Leu Ser Gly Gln Ala Leu Cys
            820                 825                 830

Asn Pro Arg Cys Arg Leu Glu Leu Arg Leu Ala Thr Ser Leu Leu Leu
        835                 840                 845

Ala Ala Pro Ser Pro Pro Val Leu Leu Leu Val Phe Leu Glu Pro Ile
    850                 855                 860

Ser Arg His Gln Leu Pro Gly Tyr His Arg Leu Ala Arg Leu Leu Arg
865                 870                 875                 880

Arg Gly Asp Tyr Cys Leu Trp Pro Glu Glu Glu Glu Arg Lys Ser Gly
                885                 890                 895

Phe Trp Thr Trp Leu Arg Ser Arg Leu Gly
            900                 905

<210>  15
<211>  991
<212>  PRT
<213>  Mus musculus

<400>  15

Met Ser Gly Leu Tyr Arg Ile Leu Val Gln Leu Glu Gln Ser Pro Tyr
1               5                   10                  15

Val Lys Thr Val Pro Leu Asn Met Arg Arg Asp Phe Phe Phe Leu Val
            20                  25                  30

Val Thr Trp Met Pro Lys Thr Val Lys Met Asn Gly Ser Ser Phe Val
        35                  40                  45

Pro Ser Leu Gln Leu Leu Leu Met Leu Val Gly Phe Ser Leu Pro Pro
    50                  55                  60

Val Ala Glu Thr Tyr Gly Phe Asn Lys Cys Thr Gln Tyr Glu Phe Asp
65                  70                  75                  80

Ile His His Val Leu Cys Ile Arg Lys Lys Ile Thr Asn Leu Thr Glu
                85                  90                  95

Ala Ile Ser Asp Ile Pro Arg Tyr Thr Thr His Leu Asn Leu Thr His
            100                 105                 110

Asn Glu Ile Gln Val Leu Pro Pro Trp Ser Phe Thr Asn Leu Ser Ala
        115                 120                 125

Leu Val Asp Leu Arg Leu Glu Trp Asn Ser Ile Trp Lys Ile Asp Glu
    130                 135                 140

Gly Ala Phe Arg Gly Leu Glu Asn Leu Thr Leu Leu Asn Leu Val Glu
145                 150                 155                 160

Asn Lys Ile Gln Ser Val Asn Asn Ser Phe Glu Gly Leu Ser Ser Leu
                165                 170                 175

Lys Thr Leu Leu Leu Ser His Asn Gln Ile Thr His Ile His Lys Asp
            180                 185                 190

Ala Phe Thr Pro Leu Ile Lys Leu Lys Tyr Leu Ser Leu Ser Arg Asn
        195                 200                 205

Asn Ile Ser Asp Phe Ser Gly Ile Leu Glu Ala Val Gln His Leu Pro
    210                 215                 220

Cys Leu Glu Arg Leu Asp Leu Thr Asn Asn Ser Ile Met Tyr Leu Asp
225                 230                 235                 240

His Ser Pro Arg Ser Leu Val Ser Leu Thr His Leu Ser Phe Glu Gly
                245                 250                 255

Asn Lys Leu Arg Glu Leu Asn Phe Ser Ala Leu Ser Leu Pro Asn Leu
            260                 265                 270

Thr Asn Leu Ser Ala Ser Arg Asn Gly Asn Lys Val Ile Gln Asn Val
        275                 280                 285

Tyr Leu Lys Thr Leu Pro Gln Leu Lys Ser Leu Asn Leu Ser Gly Thr
    290                 295                 300

Val Ile Lys Leu Glu Asn Leu Ser Ala Lys His Leu Gln Asn Leu Arg
305                 310                 315                 320

Ala Met Asp Leu Ser Asn Trp Glu Leu Arg His Gly His Leu Asp Met
                325                 330                 335

Lys Thr Val Cys His Leu Leu Gly Asn Leu Pro Lys Leu Glu Thr Leu
            340                 345                 350

Val Phe Gln Lys Asn Val Thr Asn Ala Glu Gly Ile Lys Gln Leu Ala
        355                 360                 365

Lys Cys Thr Arg Leu Leu Phe Leu Asp Leu Gly Gln Asn Ser Asp Leu
    370                 375                 380

Ile Tyr Leu Asn Asp Ser Glu Phe Asn Ala Leu Pro Ser Leu Gln Lys
385                 390                 395                 400

Leu Asn Leu Asn Lys Cys Gln Leu Ser Phe Ile Asn Asn Arg Thr Trp
                405                 410                 415

Ser Ser Leu Gln Asn Leu Thr Ser Leu Asp Leu Ser His Asn Lys Phe
            420                 425                 430

Lys Ser Phe Pro Asp Phe Ala Phe Ser Pro Leu Lys His Leu Glu Phe
        435                 440                 445

Leu Ser Leu Ser Arg Asn Pro Ile Thr Glu Leu Asn Asn Leu Ala Phe
    450                 455                 460

Ser Gly Leu Phe Ala Leu Lys Glu Leu Asn Leu Ala Ala Cys Trp Ile
465                 470                 475                 480

Val Thr Ile Asp Arg Tyr Ser Phe Thr Gln Phe Pro Asn Leu Glu Val
                485                 490                 495

Leu Asp Leu Gly Asp Asn Asn Ile Arg Thr Leu Asn His Gly Thr Phe
            500                 505                 510

Arg Pro Leu Lys Lys Leu Gln Ser Leu Ile Leu Ser His Asn Cys Leu
        515                 520                 525

Lys Ile Leu Glu Pro Asn Ser Phe Ser Gly Leu Thr Asn Leu Arg Ser
    530                 535                 540

Leu Asp Leu Met Tyr Asn Ser Leu Ser Tyr Phe His Glu His Leu Phe
545                 550                 555                 560

Ser Gly Leu Glu Lys Leu Leu Ile Leu Lys Leu Gly Phe Asn Lys Ile
                565                 570                 575

Thr Tyr Glu Thr Thr Arg Thr Leu Gln Tyr Pro Pro Phe Ile Lys Leu
            580                 585                 590

Lys Ser Leu Lys Gln Leu Asn Leu Glu Gly Gln Arg His Gly Ile Gln
        595                 600                 605

Val Val Pro Ser Asn Phe Phe Gln Gly Leu Gly Ser Leu Gln Glu Leu
    610                 615                 620

Leu Leu Gly Lys Asn Pro Ser Val Phe Leu Asp His His Gln Phe Asp
625                 630                 635                 640

Pro Leu Ile Asn Leu Thr Lys Leu Asp Ile Ser Gly Thr Lys Asp Gly
                645                 650                 655

Asp Arg Ser Leu Tyr Leu Asn Ala Ser Leu Phe Gln Asn Leu Lys Arg
            660                 665                 670

Leu Lys Ile Leu Arg Leu Glu Asn Asn Asn Leu Glu Ser Leu Val Pro
        675                 680                 685

Asp Met Phe Ser Ser Leu Gln Ser Leu Gln Val Phe Ser Leu Arg Phe
    690                 695                 700

Asn Asn Leu Lys Val Ile Asn Gln Ser His Leu Lys Asn Leu Lys Ser
705                 710                 715                 720

Leu Met Phe Phe Asp Val Tyr Gly Asn Lys Leu Gln Cys Thr Cys Asp
                725                 730                 735

Asn Leu Trp Phe Lys Asn Trp Ser Met Asn Thr Glu Glu Val His Ile
            740                 745                 750

Pro Phe Leu Arg Ser Tyr Pro Cys Gln Gln Pro Gly Ser Gln Ser Leu
        755                 760                 765

Leu Ile Asp Phe Asp Asp Ala Met Cys Asn Phe Asp Leu Gly Lys Val
    770                 775                 780

Tyr Phe Leu Cys Ser Phe Ser Met Val Leu Ser Thr Met Val Phe Ser
785                 790                 795                 800

Trp Phe Ser Thr Lys Met Ile Ala Ser Leu Trp Tyr Gly Leu Tyr Ile
                805                 810                 815

Cys Arg Ala Trp Tyr Leu Thr Lys Trp His Lys Thr Glu Lys Lys Phe
            820                 825                 830

Leu Tyr Asp Ala Phe Val Ser Phe Ser Ala Thr Asp Glu Ala Trp Val
        835                 840                 845

Tyr Lys Glu Leu Val Pro Ala Leu Glu Gln Gly Ser Gln Thr Thr Phe
    850                 855                 860

Lys Leu Cys Leu His Gln Arg Asp Phe Glu Pro Gly Ile Asp Ile Phe
865                 870                 875                 880

Glu Asn Ile Gln Asn Ala Ile Asn Thr Ser Arg Lys Thr Leu Cys Val
                885                 890                 895

Val Ser Asn His Tyr Leu His Ser Glu Trp Cys Arg Leu Glu Val Gln
            900                 905                 910

Leu Ala Ser Met Lys Met Phe Tyr Glu His Lys Asp Val Ile Ile Leu
        915                 920                 925

Ile Phe Leu Glu Glu Ile Pro Asn Tyr Lys Leu Ser Ser Tyr His Arg
    930                 935                 940

Leu Arg Lys Leu Ile Asn Lys Gln Thr Phe Ile Thr Trp Pro Asp Ser
945                 950                 955                 960

Val His Gln Gln Pro Leu Phe Trp Ala Arg Ile Arg Asn Ala Leu Gly
                965                 970                 975

Lys Glu Thr Val Glu Lys Glu Asn Thr His Leu Ile Val Val Glu
            980                 985                 990

SEQUENCES

SEQ ID NO:1

>sp|P06702|S10A9_HUMAN Calgranulin B (Migration inhibitory factor-related protein 14) (MRP- 14) (P14)
(Leukocyte L1 complex heavy chain) (S100 calcium-binding protein A9) (Calprotectin L1H subunit) - Homo
sapiens (Human).
MTCKMSQLERNIETIINTFHQYSVKLGHPDTLNQGEFKELVRKDLQNFLKKENKNEKVIEHIMEDLDTNA
DKQLSFEEFIMLMARLTWASHEKMHEGDEGPGHHHKPGLGEGTP

SEQ ID NO:2

>gi|71297360|gb|AAH26069.1| RAGE protein [Homo sapiens]
MDLWSAGCVFYEIASLQPLFPGVNELDQISKIHDVIGTPAQKILTKFKQSRAMNFDFPFKKGSGIPLLTTN
LSPQCLSLLHAMVAYDPDERIAAHQALQHPYFQEQRKTEKRALGSHRKAGFPEHPVAPEPLSNSCQISKE
GRKQKQSLKQEEDRPKRRGPAYVMELPKLKLSGVVRLSSYSSPTLQSVLGSGTNGRVPVLRPLKCIPASK
KTDPQKDLKPAPQQCRLPTIVRKGGR

SEQ ID NO:3

>gi|41350337|ref|NP_003254.2| toll-like receptor 1 [Homo sapiens]
MTSIFHFAIIFMLILQIRIQLSEESEFLVDRSKNGLIHVPKDLSQKTTILNISQNYISELWTSDILSLSK
LRILIISHNRIQYLDISVFKFNQELEYLDLSHNKLVKISCHPTVNLKHLDLSFNAFDALPICKEFGNMSQ
LKFLGLSTTHLEKSSVLPIAHLNISKVLLVLGETYGEKEDPEGLQDFNTESLHIVFPTNKEFHFILDVSV
KTVANLELSNIKCVLEDNKCSYFLSILAKLQTNPKLSNLTLNNIETTWNSFIRILQLVWHTTVWYFSISN
VKLQGQLDFRDFDYSGTSLKALSIHQVVSDVFGFPQSYIYEIFSNMNIKNFTVSGTRMVHMLCPSKISPF
LHLDFSNNLLTDTVFENCGHLTELETLILQMNQLKELSKIAEMTTQMKSLQQLDISQNSVSYDEKKGDCS
WTKSLLSLNMSSNILTDTIFRCLPPRIKVLDLHSNKIKSIPKQVVKLEALQELNVAFNSLTDLPGCGSFS
SLSVLIIDHNSVSHPSADFFQSCQKMRSIKAGDNPFQCTCELGEFVKNIDQVSSEVLEGWPDSYKCDYPE
SYRGTLLKDFHMSELSCNITLLIVTIVATMLVLAVTVTSLCSYLDLPWYLRMVCQWTQTRRRARNIPLEE
LQRNLQFHAFISYSGHDSFWVKNELLPNLEKEGMQICLHERNFVPGKSIVENIITCIEKSYKSIFVLSPN
FVQSEWCHYELYFAHHNLFHEGSNSLILILLEPIPQYSIPSSYHKLKSLMARRTYLEWPKEKSKRGLFWA
NLRAAINIKLTEQAKK

SEQ ID NO:4

>gi|20514359|gb|AAM23001.1| toll-like receptor 2 [Homo sapiens]
MPHTLWMVWVLGVIISLSKEESSNQASLSCDRNGICKGSSGSLNSIPSGLTEAVKSLDQSNNRITYICNS
DLQRCVNLQALVLTSNGINTIEEDSFSSLGSLEHLDLSYNYLSNLSSSWFKPLSSLTFLNLLGNPYKTLG
ETSLFSHLTKLQILRVGNMDTFTKIQRKDFAGLTFLEELEIDASDLQSYEPKSLKSIQNVSHLILHMKQH
ILLLEIFVDVTSSVECLELRDTDLDTFHFSELSTGETNSLIKKFTFRNVKITDESLFQVMKLLNQISGLL
ELEFDDCTLNGVGNFRASDNDRVIDPGKVETLTIRRLHIPRFYLFYDLSTLYSLTERVKRITVENSKVFL
VPCLLSQHLKSLEYLDLSENLMVEEYLKNSACEDAWPSLQTLILRQNHLASLEKTGETLLTLKNLTNIDI
SKNSFHSMPETCQWPEKMKYLNLSSTRIHSVTGCIPKTLEILDVSNNNLNLFSLNLPQLKELYISRNKLM
TLPDASLLPMLLVLKISRNAITTFSKEQLDSFHTLKTLEAGGNNFICSCEFLSFTQEQQALAKVLIDWPA
NYLCDSPSHVRGQQVQDVRLSVSECH

SEQ ID NO:5

>gi|63101664|gb|AAH94737.1| Toll-like receptor 3 [Homo sapiens]
MRQTLPCIYFWGGLLPFGMLCASSTTKCTVSHEVADCSHLKLTQVPDDLPTNITVLNLTHNQLRRLPAAN
FTRYSQLTSLDVGFNTISKLEPELCQKLPMLKVLNLQHNELSQLSDKTFAFCTNLTELHLMSNSIQKIKN
NPFVKQKNLITLDLSHNGLSSTKLGTQVQLENLQELLLSNNKIQALKSEELDIFANSSLKKLELSSNQIK
EFSPGCFHAIGRLFGLFLNNVQLGPSLTEKLCLELANTSIRNLSLSNSQLSTTSNTTFLGLKWTNLTMLD
LSYNNLNVVGNDSFAWLPQLEYFFLEYNNIQHLFSHSLHGLFNVRYLNLKRSFTKQSISLASLPKIDDFS
FQWLKCLEHLNMEDNDIPGIKSNMFTGLINLKYLSLSNSFTSLRTLTNETFVSLAHSPLHILNLTKNKIS
KIESDAFSWLGHLEVLDLGLNEIGQELTGQEWRGLENIFEIYLSYNKYLQLTRNSFALVPSLQRLMLRRV
ALKNVDSSPSPFQPLRNLTILDLSNNNIANINDDMLEGLEKLEILDLQHNNLARLWKHANPGGPIYFLKG
LSHLHILNLESNGFNEIPVEVFKDLFELKIIDLGLNNLNTLPASVFNNQVSLKSLNLQKNLITSVEKKVF
GPAFRNLTELDMRFNPFDCTCESIAWFVNWINETHTNIPELSHYLCNTPPHYHGFPVRLFDTSSCKDSA
PFELFFMINTSILLIFIFIVLLIHFEGWRISFYWNVSVHRVLGFKEIDRQTEQFEYAAYIIHAYKDKDWV
WEHFSSMEKEDQSLKFCLEERDFEAGVFELEAIVNSIKRSRKIIFVITHHLLKDPLCKRFKVHHAVQQAI
EQNLDSIILVFLEEIPDYKLNHALCLRRGMFKSHCILNWPVQKERIGAFRHKLQVALGSKNSVH

SEQ ID NO:6

>gi|109659094|gb|AAI17423.1| Toll-like receptor 4 [Homo sapiens]
MMSASRLAGTLIPAMAFLSCVRPESWEPCVEVVPNITYQCMELNFYKIPDNLPFSTKNLDLSFNPLRHLG
SYSFFSFPELQVLDLSRCEIQTIEDGAYQSLSHLSTLILTGNPIQSLALGAFSGLSSLQKLVAVETNLASLEN
FPIGHLKTLKELNVAHNLIQSFKLPEYFSNLTNLEHLDLSSNKIQSIYCTDLRVLHQMPLLNLSLDLSLNPM
NFIQPGAFKEIRLHKLTLRNNFDSLNVMKTCIQGLAGLEVHRLVLGEFRNEGNLEKFDKSALEGLCNLTIE
EFRLAYLDYYLDDIIDLFNCLTNVSSFSLVSVTIERVKDFSYNFGWQHLELVNCKFGQFPTLKLKSLKRLT
FTSNKGGNAFSEVDLPSLEFLDLSRNGLSFKGCCSQSDFGTTSLKYLDLSFNGVITMSSNFLGLEQLEHLD
FQHSNLKQMSEFSVFLSLRNLIYLDISHTHTRVAFNGIFNGLSSLEVLKMAGNSFQENFLPDIFTELRNLTF
LDLSQCQLEQLSPTAFNSLSSLQVLNMSHNNFFSLDTFPYKCLNSLQVLDYSLNHIMTSKKQELQHFPSSL
AFLNLTQNDFACTCEHQSFLQWIKDQRQLLVEVERMECATPSDKQGMPVLSLNITCQMNKTIIGVSVLS
VLVVSVVAVLVYKFYFHLMLLAGCIKYGRGENIYDAFVIYSSQDEDWVRNELVKNLEEGVPPFQLCLH
YRDFIPGVAIAANIIHEGFHKSRKVIVVVSQHFIQSRWCIFEYEIAQTWQFLSSRAGIIFIVLQKVEKTLLRQ
QVELYRLLSRNTYLEWEDSVLGRHIFWRRLRKALLDGKSWNPEGTVGTGCNWQEATSI

SEQ ID NO:7

>gi|123235038|emb|CAM28378.1| toll-like receptor 5 [Homo sapiens]














SEQ ID NO:8

>gi|119613308|gb|EAW92902.1| toll-like receptor 6 [Homo sapiens]
MTKDKEPIVKSFHFVCLMIIIVGTRIQFSDGNEFAVDKSKRGLIHVPKDLPLKTKVLDMSQNYIAELQVS
DMSFLSELTVLRLSHNRIQLLDLSVFKFNQDLEYLDLSHNQLQKISCHPIVSFRHLDLSFNDFKALPICK
EFGNLSQLNFLGLSAMKLQKLDLLPIAHLHLSYILLDLRNYYIKENETESLQILNAKTLHLVFHPTSLFA
IQVNISVNTLGCLQLTNIKLNDDNCQVFIKFLSELTRGSTLLNFTLNHIETTWKCLVRVFQFLWPKPVEY
LNIYNLTIIESIREEDFTYSKTTLKALTIEHITNQVFLFSQTALYTVFSEMNIMMLTISDTPFIHMLCPH
APSTFKFLNFTQNVFTDSIFEKCSTLVKLETLILQKNGLKDLFKVGLMTKDMPSLEILDVSWNSLESGRH
KENCTWVESIVVLNLSSNMLTDSVFRCLPPRIKVLDLHSNKIKSVPKQVVKLEALQELNVAFNSLTDLPG
CGSFSSLSVLIIDHNSVSHPSADFFQSCQKMRSIKAGDNPFQCTCELREFVKNIDQVSSEVLEGWPDSYK
CDYPESYRGSPLKDFHMSELSCNITLLIVTIGATMLVLAVTVTSLCIYLDLPWYLRMVCQWTQTRRRARN
IPLEELQRNLQFHAFISYSEHDSAWVKSELVPYLEKEDIQICLHERNFVPGKSIVENIINCIEKSYKSIF
VLSPNFVQSEWCHYELYFAHHNLFHEGSNNLILILLEPIPQNSIPNKYHKLKALMTQRTYLQWPKEKSKR
GLFWANIRAAFNMKLTLVTENNDVKS

SEQ ID NO:9

>gi|8575525|gb|AAF78035.1|AF245702_1 toll-like receptor 7 [Homo sapiens]
















SEQ ID NO:10

>gi|7576933|gb|AAF64061.1|AF246971_1 Toll-like receptor 8 [Homo sapiens]
MKESSLQNSSCSLGKETKKENMFLQSSMLTCIFLLISGSCELCAEENFSRSYPCDEKKQNDSVIAECSNR
RLQEVPQTVGKYVTELDLSDNFITHITNESFQGLQNLTKINLNHNP[?]VQHQNGNPGIQSNGLNITDGAFL
NLKNLRELLLEDNQLPQIPSGLPESLTELSLIQNNIYNITKEGISRLINLKNLYLAWNCYFNKVCEKTNI
EDGVFETLTNLELLSLSFNSLSHVSPKLPSSLRKLFLSNTQIKYISEEDFKGLINLTLLDLSGNCPRCFN
APFPCVPCDGGASINIDRFAFQNLTQLRYLNLSSTSLRKINAAWFKNMPHLKVLDLEFNYLVGEIASGAF
LTMLPRLEILDLSFNYIKGSYPQHINISRNFSKPLSLRALHLRGYVFQELREDDFQPLMQLPNLSTINLG
INFIKQIDFKLFQNFSNLEIIYLSENRISPLVKDTRQSYANSSSFQRHIRKRRSTDFEFDPHSNFYHFTR
PLIKPQCAAYGKALDLSLNSIFFIGPNQFENLPDIACLNLSANSNAQVLSGTEFSAIPHVKYLDLTNNRL
DFDNASALTELSDLEVLDLSYNSHYFRIAGVTHHLEFIQNFTNLKVLNLSHNNIYTLTDK[?]LESKSLVE
LVFSGNRLDILWNDDDNRYISIFKGLKNLTRLDLSLNRLKHIPNEAFLNLPASLTELHINDNMLKFFNWT
LLQQFPRLELLDLRGNKLLFLTDSLSDFTSSLRTLLLSHNRISHLPSGFLSEVSSLKHLDLSSNLLKTIN
KSALETKTTTKLSMLELHGNPFECTCDIGDFRRWMDEHLNVKIPRLVDVICASPGDQRGKSIVSLELTTC
VSDVTAVILFFFTFFITTMVMLAALAHHLFYWD[?]WFIYNVCLAKIKGYRSLSTSQTFYDAYISYDTKDAS
VTDWVINELRYHLEESRDKNVLLCLEERDWDPGLAIIDNLMQSINQSKKTVFVLTKKYAKSWNFKTAFYL
ALQRLMDENMDVIIFILLEPVLQHSQYLRLRQRICKSSILQWPDNPKAEGLFWQTLRNVVLTENDSRYNN
MYVDSIKQY

SEQ ID NO:11

>gi|8575529|gb|AAF78037.1|AF245704_1 toll-like receptor 9 [Homo sapiens]
MGFCRSALHPLSLLVQAIMLAMTLALGTLPAFLPCELQPHGLVNCNWLFLKSVPHFSMAAPRGNVTSLSL
SSNRIHHLHDSDFAHLPSLRHLNLKWNCPPVGLSPMHFPCHMTIEPSTFLAVPTLEELNLSYNNIMTVPA
LPKSLISLSLSHTNILMLDSASLAGLHALRFLFMDGNCYYKNPCRQALEVAPGALLGLGNLTHLSLKYNN
LTVVPRNLPSSLEYLLLSYNRIVKLAPEDLANLTALRVLDVGGNCRRCDHAPNPCMECPRHFPQLHPDTF
SHLSRLEGLVLKDSSLSWLNASWFRGLGNLRVLDLSENFLYKCITKTKAFQGLTQLRKLNLSFNYQKRVS
FAHLSLAPSFGSLVALKELDMHGIFFRSLDETTLRPLARLPMLQTLRLQMNFINQAQLGIFRAFPGLRYV
DLSDNRISGASELTATMGEADGGEKVWLQPGDLAPAPVDTPSSEDFRPNCSTLNFTLDLSRNN[?]TVQPE
MFAQLSHLQCLRLSHNCISQAVNGSQFLPLTGLQVLDLSRNKLDLYHEHSFTELPRLEALDLSYNSQPFG
MQGVGHNFSFVAHLRTLRHLSLAHNNIHSQVSQQLCSTSLRALDFSGNALGHMWAEGDLYLHFFQGLSGL
IWLDLSQNRLHTLLPQTLRNLPKSLQVLRLRDNYLAFFKWWSLHFLPKLEVLDLAGNRLKALTNGSLPAG
TRLRRLDVSCNSISFVAPGFFSKAKELRELNLSANALKTVDHSWFGPLASALQILDVSANPLHCACGAAF
MDFLLEVQAAVPGLPSRVKCGSPGQLQGLSIFAQDLRLCLDEALSWDCFALSLLAVALGLGVPMLHHLCG
WDLWYCFHLCLAWLPWRGRQSGRDEDALPYDAFVVFDKTQSAVADWVYNELRGQLEECRGRWALRLCLEE
RDWLPGKTLFENLWASVYGSRKTLFVLAHTDRVSGLLRASFLLAQQRLLEDRKDVVVLVILSPDGRRSRY
VRLRQRLCRQSVLLWPHQPSGQRSFWAQLGMALTRDNHHFYNRNFCQGPTAE

SEQ ID NO:12

>gi|13447753|gb|AAK26744.1|AF296673_1 toll-like receptor 10 [Homo sapiens]
MRLIRNIYIFCSIVMTAEGDAPELPEERELMTNCSNMSLRKVPADLTPATTTLDLSYNLLFQLQSSDFHS
VSKLRVLILCHNRIQQLDLKTFEFNKELRYLDLSNNRLKSVTWYLLAGL[?]LDLSFNDFDTMPICEEAGN
MSHLEILGLSGAKIQKSDFQKIAHLHLNTVFLGFRTLPHYEEGSLPILNTTKLHIVLPMDTNFWVLLRDG
IKTSKILEMTNIDGKSQFVSYEMQRNLSLENAKTSVLLLNKVDLLWDDLFLILQFVWHTSVEHFQIRNVT
FGGKAYLDHNSFDYSNTVMRTIKLEHVHFRVFYIQQDKIYLLLTKMDIENLTISNAQMPHMLFPNYPTKF
QYLNFANNILTDELFKRTIQLPHLKTLILNGNKLETLSLVSCFANNTPLEHLDLSQNLLQHKNDENCSWP
ETVVNMNLSYNKLSDSVFRCLPKSIQILDLNNNQIQTVPKETIHLMALRELNIAFNFLTDLPGCSHFSRL
SVLNIEMNFILSPSLDFVQSCQEVKTLNAGRNPFRCTCELKNFIQLETYSEVMMVGWSDSYTCEYPLNLR
GIRLKDVHLHELSCNTALLIVTIVVIMLVLGLAVAFCCLHFDLPWYLRMLGQCTQTWHRVRKTT[?]QLKR
NVRFHAFISYSEHDSLWVKNELIPNLEKEDGSILICLYESYFDPGKSISENIVSFIEKSYKSIFVLSPNF
VQNEWCHYEFYFAHHNLFHENSDHIILILLEPIPFYCIPTRYHKLKALLEKKAYLEWPKDRRKCGLFWAN
LRAAINVNVLATREMYELQTFTELNEESRGSTISLMRTDCL

SEQ ID NO: 13

>gi|80478199|gb|AAI09165.1| Toll-like receptor 11 [Mus musculus]
MPRMERHQFCSVLLILILLTLVSLTLTGWAWTIPDCIIADSLLFPNLSYYIPFCTSAPGLHLLASCSNVK
NLNQTLKRVPRNTEVLCLQGMVPTLPAKAFIRFHSLQLLRLQLSTTSVTSRTFQGLDQLQYLFFDHHAPC
CLSLFLSPNCFESLRSLSSLSFQGYCLTYSQSIYLPTSLRHLTLRNSCLTKFQDLQRLFPDLLLSTSSTP
NTKPGAPFLEMLDLSYNLQLKQAGVRDIYGLTLHSLILDGTPLRALDLTDSGLLHLHFLSLVGTGIEKVP
ASLTGYSELRALDLGKNQIQNILENGEIPGYKALEFLSLHDNHLQTLPTRFLHTLPQLQKLNLSMNKLGP
ILELPEGLFSTNLKVLDLSHNQLCDVPHGAFSLLSQLQELWLSGNNISSLSNESLQGLRQLRTLDLSWNQ
IKVLKPGWLSHLPALTTLNLLGTYLENILGIQLQGPKMLRHLQLGSYPMLDIYPPWPPTLLSLEIQAESC
IQFMIHSGQPFLFLENLTLETSILLLKPDNITIHFPSLRRLTLRGYSFIFSTSQLQRFFPQQLPLLEHFF
IWCENSYAVDLYLFGMPRLRVLELGYLNFFYESSTMKLEMLLKEVPQLQVLALSHLNLRNLSVSSFKSLQ
DLKLLLFNSERALEMNSNLQEFIPQMPQYVYFSDVTFTCQCEASWLESWATRAPNTFVYGLEKSICIANA

SDYSKTLLFSFLATNCPHGTEFWGFLTSFILLLLLLIILPLIS@KWSWLHHLWTLFHTCWWKLCGHRLRG
QFNYDVFISYCEEDQAWVLEELVPVLEKAPPEGEGLRLCLPARDFGIGNDRMESMIASMGKSRATLCVLT
GQALASPWCNLELRLATYHLVARPGTTHLLLLFLEPLDRQRLHSYHRLSRWLQKEDYFDLSQGKVEWNSF
CEQLKRRLSKAGQERD

SEQ ID NO:14

>gi|124297779|gb|AAI31922.1| Toll-like receptor 12 [Mus musculus]
MGRYWLLPGLLLSLPLVTGWSTSNCLVTEGSRLPLVSRYFTFCRHSKLSFLAACLSVSNLTQTLEVVPRT
VEGLCLGGTVSTLLPDAFSAFPGLKVLALSLHLTQLLPGALRGLGQLQSLSFFDSPLRRSLFLPPDAFSD
LISLQRLHISGPCLDKKAGIRLPPGLQWLGVTLSCIQDVGELAGMFPDLVQGSSSRVSWTLQKLDLSSNW
KLKMASPGSLQGLQVEILDLTRTPLDAVWLKGLGLQKLDVLYAQTATAELAAEAVAHFELQGLIVKESKI
GSISQEALASCHSLKTLGLSSTGLTKLPPGFLTAMPRLQRLELSGNQLQSAVLCMNETGDVSGLTTLDLS
GNRLRILPPAAFSCLPHLRELLLRYNQLLSLEGYLFQELQQLETLKLDGNPLLHLGKNWLAALPALTTLS
LLDTQIRMSPEPGFWGAKNLHTLSLKLPALPAPAVLFLPMYLTSLELHIASGTTEHWTLSPAIFPSLETL
TISGGGLKLKLGSQNASGVFPALQKLSLLKNSLDAFCSQGTSNLFLWQLPKLQSLRVWGAGNSSRPCLIT
GLPSLRELKLASLQSITQPRSVQLEELVGDLPQLQALVLSSTGLKSLSAAAFQRLHSLQVLVLEYEKDLM
LQDSLREYSPQMPHYIYILESNLACHCANAWMEPWVKRSTKTYIYIRDNRLCPGQDRLSARGSLPSFLWD
HCPQTLELKLFLASSALVFMLIALPLLQEARNSWIPYLQALFRVWQGLRGKGDKGKRFLFDVFVSHCRQ
DQGWVIEELLPALEGFLPAGLGLRLCLPERDFEPGKDVVDNVVDSMLSSRTTLCVLSGQALCNPRCRLEL
RLATSLLLAAPSPPVLLLVFLEPISRHQLPGYHRLARLLRRGDYCLWPEEEERKSGFWTWLRSRLG

SEQ ID NO:15

>gi|109734930|gb|AAI17914.1| Toll-like receptor 13 [Mus musculus]
MSGLYRILVQLEQSPYVKTVPLNMRRDFFFLVVTWMPKTVKMNGSSFVPSLQLLLMLVGFSLPPVAETYG
FNKCTQYEFDIHHVLCIRKKITNLTEAISDIPRYTTHLNLTHNEIQVLPPWSFTNLSALVDLRLEWNSIW
KIDEGAFRGLENLTLLNLVENKIQSVNNSFEGLSSLKTLLLSHNQITHIHKDAFTPLIKLKYLSLSRNNI
SDFSGILEAVQHLPCLERLDLTNNSIMYLDHSPRSLVSLTHLSFEGNKLRELNFSALSLPNLTNLSASRN
GNKVIQNVYLKTLPQLKSLNLSGTVIKLENLSAKHLQNLRAMDLSNWELRHGHLDMKTVCHLLGNLPKLE
TLVFQKNVTNAEGIKQLAKCTRLLFLDLGQNSDLIYLNDSEFNALPSLQKLNLNKCQLSFINNRTWSSLQ
NLTSLDLSHNKFKSFPDFAFSPLKHLEFLSLSRNPITELNNLAFSGLFALKELNLAACWIVTIDRYSFTQ
FPNLEVLDLGDNNIRTLNHGTFRPLKKLQSLILSHNCLKILEPNSFSGLTNLRSLDLMYNSLSYFHEHLF
SGLEKLLILKLGFNKITYETTRTLQYPPFIKLKSLKQLNLEGQRHGIQVVPSNFFQGLGSLQELLLGKNP
SVFLDHHQFDPLINLTKLDISGTKDGDRSLYLNASLFQNLKRLKILRLENNNLESLVPDMFSSLQSLQVF
SLRFNNLKVINQSHLKNLKSLMFFDVYGNKLQCTCDNLWFKNWSMNTEEVHIPFLRSYPCQQPGSQSLLI
DFDDAMCNFDLGKVYFLCSFSMVLSTMVFSWFSTKMIASLWYGLYICRAWYLTKWHKTEKKFLYDAFVSF
SATDEAWVYKELVPALEQGSQTTFKLCLHQRDFEPGIDIFENIQNAINTSRKTLCVVSNHYLHSEWCRLE
VQLASMKMFYEHKDVIILIFLEEIPNYKLSSYHRLRKLINKQTFITWPDSVHQQPLFWARIRNALGKETV
EKENTHLIVVE

## Claims

1. A method of identifying a compound for use in therapy which modulates the interaction of S100A9 with a ligand comprising:
(i) determining whether a candidate compound is capable of modulating the interaction of S100A9 with RAGE or the interaction of S100A9 with a TLR (Toll like receptor), or
(ii) determining whether a candidate compound is capable of binding to S100A9 in a manner which disrupts binding of S100A9 with RAGE or the binding of S100A9 with a TLR,
to thereby identify whether the compound may be used in therapy.

2. A method according to claim 1 (ii) wherein said determining is carried out by measuring whether the candidate compound binds the site on S100A9 which is bound by RAGE or the site on S100A9 which is bound by a TLR.

3. A method according to claim 1 or 2 which tests whether the compound is capable of inhibiting the interaction of S100A9 with RAGE or a TLR.

4. A method according to any one of the preceding claims wherein the therapy is cancer therapy or immunotherapy and/or wherein the TLR is TLR4.

5. A method according to any one of the preceding claims wherein:
(i) said ligand and/or S100A9 are present in isolated, and/or one of the said ligand or S100A9 are immobilized to a solid surface, or
(ii) during the method the effect of the candidate substance on the binding of the ligand to S100A9 is measured over at least 60 to 1200 seconds, preferably 120 to 400 seconds, to allow the kinetics of binding to be observed, or
(iii) the dose dependency of the candidate compound on the binding between the ligand and S100A9 is measured, or
(iv) the detection of binding is carried out in the presence of calcium and zinc ions.

6. A method according any one of the preceding claims which comprises
(i) contacting the candidate compound with S100A9 and determining whether the compound is able to inhibit binding of said S100A9 with RAGE, or
(ii) contacting the candidate compound with RAGE and determining whether the compound is able to inhibit binding of said RAGE with S100A9, or
(iii) contacting the candidate compound a S100A9/RAGE complex and determining whether the candidate compound causes dissociation of the complex.

7. A method according to any one of the preceding claims wherein a fragment and/or homologue of S100A9 and/or RAGE and/or a TLR4 is used, which fragment or homologue is optionally in the form of a fusion protein.

8. A method according to any one of the preceding claims wherein:
(i) a fragment and/or homologue of S100A9 is used which is capable of binding RAGE and/or zinc, and/or
(ii) a fragment and/or homologue of RAGE or a TLR is used which is capable of binding S100A9, and/or
(iii) RAGE or TLR are glycosylated, optionally by expressing them in a mammalian cell.

9. A method according to any one of the preceding claims wherein detection of binding is carried out in conditions in which RAGE and/or TLR4 would not bind to other S100 proteins, and optionally in conditions in which RAGE and/or TLR4 would not bind to S100A1, S100A12, S100B or S100P.

10. A method according to any one of the preceding claims which determines whether:
(i) the compound fits a 1:1 model of binding (one site-competition model), or
(ii) the compound has an IC50 of between 1nM to 200uM, or
(iii) the compounds causes inhibition as shown in Figure 7A, 7B or 7C, or
(iv) the compound decreases the on-rate for the binding of S100A9 to the ligand,
and optionally the compound is selected based on (i), (ii), (iii) or (iv).

11. A method according to any one of the preceding claims in which:
(i) S100A9 is present in the form of a monomer, dimer or multimer that consists only of S100A9 and/or in the form of a S100A8/S100A9 complex, and/or
(ii) the ligand is present in the form of a monomer or dimer

12. A method according to any one of the preceding claims wherein:
(i) the identified compound is further tested to determine whether it reduces an immune response, optionally using a cell based assay or an animal model, and/or
(ii) wherein at least 100,000 different compounds are tested by the method of any one of claims 1 to 11 and of these at least 10,000 are further tested to determine whether they reduce an immune response.

13. A method according to claim 12 wherein the cell based assay comprises monocytes which express S100A9, and is preferably a mixed lymphocyte reaction.

14. A method according to any of the preceding claims further comprising determining whether the identified compound has therapeutic efficacy in an animal model, wherein optionally the animal model is a cancer model, an autoimmune disease model or a model of an inflammatory condition.

15. A method according to any one of the preceding claims further comprising formulating the identified compound into a pharmaceutical composition.

16. A method of therapy comprising identifying a compound by a method according to any of claims 1 to 14 and administering the identified compound to an individual in need of therapy.
